# EUROPÄISCHE PATENTSCHRIFT

(11) **EP 2 229 110 B1**
(45) Veröffentlichungstag und Bekanntmachung des Hinweises auf die Patenterteilung: **09.11.2016**
(21) Anmeldenummer: 08858333.1
(22) Anmeldetag: 03.12.2008
(51) Int. Cl.: A61B 17/16

(54) **AUFSATZVORRICHTUNG FÜR EINE CHIRURGISCHE VORRICHTUNG SOWIE CHIRURGISCHE VORRICHTUNG ZUM DURCHSCHNEIDEN EINES KNOCHENS**
ATTACHMENT DEVICE FOR A SURGICAL DEVICE AND SURGICAL DEVICE FOR CUTTING THROUGH A BONE
DISPOSITIF RAPPORTÉ POUR DISPOSITIF CHIRURGICAL ET DISPOSITIF CHIRURGICAL CORRESPONDANT POUR PERCER UN OS

(30) Priorität: 05.12.2007 DE 102007060493
(43) Veröffentlichungstag der Anmeldung: 22.09.2010
(73) Patentinhaber: Aesculap AG, 78532 Tuttlingen (DE)
(72) Erfinder: LUTZE, Theodor, 78582 Balgheim (DE); HÄUSLER, Rainer, 78532 Tuttlingen (DE); HERMANN, Reiner, 78567 Fridingen (DE)
(74) Vertreter: Hoeger, Stellrecht & Partner Patentanwälte mbB
(86) Internationale Anmeldenummer: PCT/EP2008/066704
(87) Internationale Veröffentlichungsnummer: WO 2009/071581

(56) Entgegenhaltungen:
- DE-A1- 19 742 535
- DE-B3- 10 240 655
- DE-C- 424 100
- DE-U1-202004 006 659
- DE-U1-202007 017 509
- US-A- 5 876 405

## Beschreibung

Die Erfindung betrifft eine Aufsatzvorrichtung für eine chirurgische Vorrichtung, wobei die Aufsatzvorrichtung eine Schneideinrichtung mit mindestens einem mindestens eine Schneidkante bildenden Schneidelement zur Erzeugung mindestens einer Schnittlinie an einem Knochen umfasst, wobei die Aufsatzvorrichtung eine Vorschubeinrichtung aufweist, die mindestens ein Vorschubglied umfasst, welches relativ zur mindestens einen Schneidkante beweglich ausgebildet ist und dadurch relativ zu dieser einen variablen Abstand einnehmen kann, sowie eine Kraftbeaufschlagungseinrichtung, welche so ausgebildet ist, dass das mindestens eine Vorschubglied mit einer in distaler Richtung wirkenden Kraft beaufschlagbar ist, zumindest sofern der Abstand einen Mindestabstand überschreitet, und wobei die Aufsatzvorrichtung eine Abstützeinrichtung aufweist mit mindestens einem an den Knochen anlegbaren Abstützglied.

Darüber hinaus betrifft die Erfindung eine chirurgische Vorrichtung zum Durchschneiden eines Knochens mit mindestens einer Aufsatzvorrichtung.

Eine Aufsatzvorrichtung der eingangs genannten Art kann als Zubehör oder Ergänzungsvorrichtung für die letztgenannte chirurgische Vorrichtung zum Durchschneiden eines Knochens zum Einsatz kommen oder einen Bestandteil, insbesondere eine Baueinheit, derselben bilden.

Unter Einsatz einer bekannten Aufsatzvorrichtung kann ein Knochen, beispielsweise der menschliche Schädel, durchgeschnitten werden, etwa zur Erzeugung einer Öffnung im Schädel, über welche ein Operateur chirurgische Instrumente in das Körperinnere eines Patienten einführen kann. Nachfolgend wird vereinfachend und nicht-limitierend für die Anwendbarkeit der Aufsatzvorrichtung und der chirurgischen Vorrichtung angenommen, dass diese am menschlichen Schädel zum Einsatz kommen, wobei sie auch zum Durchschneiden anderer Knochen verwendet werden können. Dementsprechend bezieht sich nachfolgend "Körperinneres des Patienten" nicht nur auf das Innere des menschlichen Schädels, sondern allgemein auf dem zu durchschneidenden Knochen benachbarte Bereiche des Körpers.

Bei bekannten Aufsatzvorrichtungen hat es sich als nachteilig für die Handhabung erwiesen, dass für den Operateur oftmals nicht zu erkennen ist, wann der Knochen durchgeschnitten ist. Deswegen muss der Operateur üblicherweise unter Einsatz langsamen Vortriebs des mindestens einen Schneidelementes und größter Vorsicht beim Schneidvorgang vorgehen, um jenseits des Knochens liegendes Körpergewebe, zum Beispiel Weichgewebe oder eine Knochenhaut, beim Durchschneiden des Knochens nicht mittels des mindestens einen Schneidelementes zu verletzen.

Vorrichtungen zum Durchschneiden eines Knochens mit den Merkmalen einer Aufsatzvorrichtung der eingangs genannten Art sind in den Druckschriften US 5,876,405 A und DE 197 42 535 A1 beschrieben. Eine weitere Vorrichtung zum Durchschneiden eines Knochens ist in der DE 42 41 00 C beschrieben.

Aufgabe der vorliegenden Erfindung ist es, eine Aufsatzvorrichtung sowie eine chirurgische Vorrichtung der eingangs genannten Art bereitzustellen, mit der ein Verletzungsrisiko für den Patienten verringerbar ist.

Diese Aufgabe wird bei einer gattungsgemäßen Aufsatzvorrichtung erfindungsgemäß dadurch gelöst, dass die Abstützeinrichtung verdrehgesichert zur Vorschubeinrichtung ausgebildet ist.

Das mindestens eine Vorschubglied der Vorschubeinrichtung ist relativ zur mindestens einen Schneidkante beweglich ausgebildet. Infolgedessen kann das mindestens eine Vorschubglied relativ zur mindestens einen Schneidkante einen variablen Abstand einnehmen. Beispielsweise kann eine Relativbewegung des mindestens einen Vorschubgliedes und der mindestens einen Schneidkante und eine damit verbundene Abstandsänderung durch einen Vortrieb des mindestens einen Schneidelementes während des Schneidvorganges in den Knochen erzielt werden. Auf diese Weise kann der Abstand einen vorgebbaren Mindestabstand überschreiten, welcher etwa durch die Dicke des zu durchschneidenden Knochens vorgebbar oder mittels dieser bestimmbar ist. Dementsprechend kann das mindestens eine Vorschubglied relativ zur mindestens einen Schneidkante dann den Mindestabstand aufweisen, wenn der Knochen durchgeschnitten ist.

Weiter ist vorgesehen, dass zumindest dann, wenn der Abstand einen Mindestabstand überschreitet, das mindestens eine Vorschubglied durch die Kraftbeaufschlagungseinrichtung mit einer in distaler Richtung wirkenden Kraft beaufschlagbar ist. "Distal" ist vorliegend in Bezug auf den die Aufsatzvorrichtung einsetzenden Operateur aufzufassen. Wird die Aufsatzvorrichtung wie üblich mit mindestens einer Hand geführt, bedeutet "distal" vorliegend "in von der Hand des Operateurs wegweisender Richtung".

Durch die Kraftbeaufschlagung kann das mindestens eine Vorschubglied in distaler Richtung bewegt werden. Weiter wird ermöglicht, den zu durchschneidenden Knochen mittels des mindestens einen Vorschubgliedes mit einer Kraft zu beaufschlagen und diesen dadurch ebenfalls in distaler Richtung zu bewegen, insbesondere in das Körperinnere des Patienten. Jenseits des Knochens liegendes Gewebe, beispielsweise eine Knochenhaut, kann dadurch ebenfalls in distaler Richtung bewegt werden und somit weg von dem mindestens einen Schneidelement. Dies erlaubt es bei sachgemäßer Handhabung der Aufsatzvorrichtung, ein Verletzungsrisiko des hinter dem Knochen liegenden Gewebes zu verringern. Zudem wird zugleich die Handhabbarkeit der Aufsatzvorrichtung verbessert.

Um zu ermitteln, ob der Abstand des mindestens einen Vorschubgliedes von der mindestens einen Schneidkante den erforderlichen Mindestabstand überschreitet, kann die Aufsatzvorrichtung eine Detektionseinrichtung umfassen. Diese kann auf vielfältige Weise ausgebildet sein, beispielsweise mechanisch und/oder optisch. Zum Beispiel ist es möglich, dass mittels der Detektionseinrichtung der Widerstand, den der zu durchschneidende Knochen dem mindestens einen Schneidelement während des Schneidvorganges entgegensetzt, erfasst wird. Dessen Abfall kann ein Indiz dafür sein, dass der Knochen durchgeschnitten ist.

Alternativ kann die Detektionseinrichtung beispielsweise ein Detektionsglied umfassen, das den Knochen bereits während des Schneidvorganges mit einer Kraft beaufschlagt, welche durch eine Gegenkraft des Knochens kompensiert wird. Ist der Knochen durchgeschnitten, kann dessen Gegenkraft entfallen, da jenseits des Knochens angeordnetes Gewebe weicher als der Knochen ausgebildet ist. Das Entfallen der Gegenkraft ist mittels des Detektionsgliedes erfassbar und beispielsweise zur Aktivierung der Kraftbeaufschlagungseinrichtung nutzbar.

Die erfindungsgemäße Aufsatzvorrichtung weist außerdem eine Abstützeinrichtung auf mit mindestens einem an den Knochen anlegbaren Abstützglied. Mittels der Abstützeinrichtung kann der Operateur die Aufsatzvorrichtung am Knochen abstützen. Dies kann insbesondere auf eine solche Weise erfolgen, dass dadurch verhindert wird, dass der Operateur die Aufsatzvorrichtung nach dem Durchschneiden des Knochens als Ganzes in das Körperinnere des Patienten hineindrückt. Eine Verletzungsgefahr des sich hinter dem zu durchschneidenden Knochen befindenden Körpergewebes lässt sich dadurch weiter verringern.

Ferner ist die Abstützeinrichtung verdrehgesichert zur Vorschubeinrichtung ausgebildet. Dies ist insbesondere dann vorteilhaft, wenn die Schneideinrichtung drehend angetrieben wird und sowohl relativ zur Vorschubeinrichtung als auch relativ zur Abstützeinrichtung drehbeweglich ausgebildet ist. Letztere können dann jeweils mit dem Knochen in Eingriff stehen und verdrehgesichert relativ zu diesem ausgebildet sein. Dies hat sich als besonders vorteilhaft für die Handhabbarkeit der Aufsatzvorrichtung und für die Zuverlässigkeit ihres Betriebs erwiesen.

Günstig ist es, wenn die Kraftbeaufschlagungseinrichtung so ausgebildet ist, dass das mindestens eine Vorschubglied unabhängig von seinem Abstand von der mindestens einen Schneidkante mit einer in distaler Richtung wirkenden Kraft beaufschlagbar ist. Dies erlaubt es, der Aufsatzvorrichtung eine einfache und zuverlässige Funktionsweise zu verleihen. Die Kraftbeaufschlagungseinrichtung kann dadurch rasch auf ein Überschreiten des Mindestabstandes reagieren, da das mindestens eine Vorschubglied dauerhaft mit der Kraft beaufschlagbar ist. Insbesondere ermöglicht es diese Ausführungsform, das mindestens eine Vorschubglied als das vorstehend beschriebene Detektionsglied auszubilden, welches den zu durchschneidenden Knochen mit der Kraft beaufschlagt, deren Gegenkraft nach dem Durchschneiden des Knochens entfällt. Nach dem Entfallen der Gegenkraft des Knochens kann dieser, etwa unter Verdrängung von jenseits des Knochens angeordnetem Gewebe, mittels des mindestens einen Vorschubgliedes in distaler Richtung bewegt werden.

Von Vorteil ist es, wenn das mindestens eine Vorschubglied eine Anlagefläche zum Anlegen des mindestens einen Vorschubgliedes an den Knochen aufweist. Dies gibt die Möglichkeit, den Knochen über das mindestens eine Vorschubglied mit einer in distaler Richtung wirkenden Kraft zu beaufschlagen. Bildet das mindestens eine Vorschubglied das vorstehend beschriebene Detektionsglied, kann mittels der Anlagefläche das Vorhandensein und das Entfallen einer Gegenkraft des Knochens erfasst werden.

Bevorzugt umfasst das mindestens eine Vorschubglied mindestens ein sich in distaler Richtung erstreckendes Eingriffselement zum Eingreifen in den Knochen. Dadurch kann ein Eingriff zwischen dem mindestens einen Vorschubglied und dem Knochen sichergestellt werden. Bei einer Kraftbeaufschlagung des mindestens einen Vorschubgliedes wird somit der Knochen ebenfalls zuverlässig kraftbeaufschlagt. Es kann vorgesehen sein, dass das mindestens eine Vorschubglied über das mindestens eine Eingriffselement hinaus die vorstehend beschriebene Anlagefläche zum Anlegen an den Knochen aufweist.

Die Kraftbeaufschlagungseinrichtung kann auf vielfältige Weise ausgebildet sein. Insbesondere kann sie mittelbar oder unmittelbar auf das mindestens eine Vorschubglied einwirken. Ein Beispiel für eine mittelbar auf das mindestens eine Vorschubglied einwirkende Kraftbeaufschlagungseinrichtung ist eine solche, die auf das mindestens eine Vorschubglied unter Einsatz eines Mediums wie beispielsweise eines Fluids einwirkt. So kann die Kraftbeaufschlagungseinrichtung eine Düse umfassen, aus der zur Kraftbeaufschlagung des mindestens einen Vorschubgliedes ein Fluid wie Pressluft ausströmt, wenn der Abstand des mindestens einen Vorschubgliedes von der mindestens einen Schneidkante den Mindestabstand überschreitet.

Eine unmittelbar auf das mindestens eine Vorschubglied einwirkende Kraftbeaufschlagungseinrichtung kann auf dieses in körperlicher Form einwirken. Dies erfolgt vorzugsweise dadurch, dass die Kraftbeaufschlagungseinrichtung an dem mindestens einen Vorschubglied anliegt. Dies gibt auf technisch einfache Weise die Möglichkeit, das mindestens eine Vorschubglied mit der Kraft in distaler Richtung zu beaufschlagen.

Günstig ist es, wenn die Kraftbeaufschlagungseinrichtung ein elastisches Element zur Kraftbeaufschlagung des mindestens einen Vorschubgliedes umfasst, denn dies ermöglicht eine konstruktiv einfache Ausgestaltung der Aufsatzvorrichtung. Das elastische Element, das vorteilhafterweise an dem mindestens einen Vorschubglied anliegt, umfasst zum Beispiel Federn jedweder Art wie Spiralfedern, Gasdruckfedern, Blattfedern, elastische Gummimanschetten oder dergleichen.

Das mindestens eine Vorschubglied kann mittels der Kraftbeaufschlagungseinrichtung unabhängig von seinem Abstand von der mindestens einen Schneidkante auf technisch einfache Weise mit einer in distaler Richtung wirkenden Kraft beaufschlagt werden, wenn das elastische Element relativ zum mindestens einen Vorschubglied vorgespannt ist.

Vorzugsweise definiert die Aufsatzvorrichtung eine Achse, entlang derer das mindestens eine Vorschubglied relativ zur mindestens einen Schneidkante beweglich ausgebildet ist. Dies erlaubt es, eine definierte Bewegung des mindestens einen Vorschubgliedes sicherzustellen und sorgt für eine zuverlässige Funktionsweise der Aufsatzvorrichtung.

Günstig ist es, wenn die Aufsatzvorrichtung eine Führung für das mindestens eine Vorschubglied zu dessen Bewegung relativ zur mindestens einen Schneidkante aufweist. Damit lässt sich die Bewegung des mindestens einen Vorschubgliedes relativ zur mindestens einen Schneidkante noch zuverlässiger ausbilden. Zur Bildung der Führung kann die Aufsatzvorrichtung einen Führungskörper aufweisen, der beispielsweise an der Schneideinrichtung oder der Vorschubeinrichtung angeordnet ist oder von dieser umfasst wird.

Um eine Bewegung des mindestens einen Vorschubgliedes relativ zur mindestens einen Schneidkante entlang einer Achse der Aufsatzvorrichtung auf technisch einfache Weise sicherzustellen, ist das mindestens eine Vorschubglied relativ zur mindestens einen Schneidkante vorzugsweise verschiebbar geführt, zum Beispiel den vorstehend genannten Führungskörper entlang.

Von Vorteil ist es, wenn dem mindestens einen Vorschubglied zur Begrenzung dessen Bewegung relativ zur mindestens einen Schneidkante mindestens ein Anschlag zugeordnet ist. Mittels des mindestens einen Anschlages, der zum Beispiel an der Schneideinrichtung und/oder der Vorschubrichtung angeordnet sein kann oder davon umfasst wird, lässt sich der Bewegungsumfang des mindestens einen Vorschubgliedes begrenzen.

Es kann vorgesehen sein, dass der mindestens eine Anschlag zur Begrenzung der Bewegung des mindestens einen Vorschubgliedes in distaler Richtung ausgebildet ist. Wird die Bewegung des mindestens einen Vorschubgliedes in distaler Richtung begrenzt, ist dadurch die Möglichkeit gegeben, auch eine Bewegung des Knochens, welcher mittels des mindestens einen Vorschubgliedes kraftbeaufschlagt wird, in distaler Richtung zu begrenzen. Somit kann der Vorschub des Knochens in das Körperinnere des Patienten begrenzt werden. Dies erlaubt es, das sich jenseits des Knochens befindende Körpergewebe nur so weit von der Schneidkante zu entfernen, dass die Verletzungsgefahr des Körpergewebes durch das mindestens eine Schneidelement reduziert wird und zugleich eine Verletzungsgefahr, die durch Stauchung des Körpergewebes durch den Vorschub des Knochens bestehen könnte, zu verringern. Auf konstruktiv einfache Weise lässt sich die Bewegung des mindestens einen Vorschubgliedes in distaler Richtung beispielsweise dadurch begrenzen, dass das mindestens eine Vorschubglied zumindest teilweise zwischen dem mindestens einen Anschlag und der Kraftbeaufschlagungseinrichtung angeordnet ist.

In ähnlicher Weise kann vorgesehen sein, dass der mindestens eine Anschlag zur Begrenzung der Bewegung des mindestens einen Vorschubgliedes in proximaler Richtung ausgebildet ist, wobei "proximal" vorliegend entgegengesetzt zu "distal" aufzufassen ist. Beispielsweise lässt sich dies auf konstruktiv einfache Weise dadurch erzielen, dass die Kraftbeaufschlagungseinrichtung zumindest teilweise zwischen dem Anschlag und dem mindestens einen Vorschubglied angeordnet ist. Dabei kann sich das mindestens eine Vorschubglied über die Kraftbeaufschlagungseinrichtung an dem mindestens einen Anschlag abstützen oder daran anliegen.

Von Vorteil ist, wenn die Schneideinrichtung so ausgebildet ist, dass die mindestens eine Schnittlinie in sich geschlossen oder im Wesentlichen in sich geschlossen erzeugbar ist. Dadurch lässt sich auf technisch einfache Weise aus dem Knochen ein Knochensegment ausschneiden, das eine in sich geschlossene Randlinie aufweist. Nach Entfernen eines derartigen Knochensegmentes weist der Knochen somit eine Öffnung auf, durch die der Operateur Instrumente ins Körperinnere einführen kann. Günstigerweise ist die Schnittlinie rund und noch günstigerweise kreisförmig erzeugbar.

Auf konstruktiv einfache Weise lässt sich die vorstehend beschriebene Schnittlinie dadurch erzeugen, dass die Schneideinrichtung einen zumindest teilweise hülsenartigen Schneidkörper umfasst und dass das mindestens eine Schneidelement an einer dessen Enden angeordnet ist. Der Schneidkörper kann zum Beispiel hohlzylindrisch ausgebildet sein, so dass das mindestens eine Schneidelement beim Durchschneiden des Knochen eine kreisrunde und in sich geschlossene Schnittlinie erzeugen kann etwa dann, wenn die Schneideinrichtung rotierend antreibbar ist. Die Schneideinrichtung kann, um angetrieben zu werden, beispielsweise an dem dem mindestens einen Schneidelement entgegensetzten Ende des Schneidkörpers Mittel zur Verbindung mit einer Antriebseinrichtung aufweisen.

Eine kompakte Bauform und ein zuverlässiger Betrieb der Aufsatzvorrichtung, insbesondere bei zuletzt beschriebener Ausführungsform, lässt sich dadurch erzielen, dass die Schneideinrichtung koaxial zur Vorschubeinrichtung ausgerichtet ist.

Eine kompakte Bauform der Aufsatzvorrichtung kann auch dadurch erzielt werden, dass die Schneideinrichtung, beispielsweise mit dem vorstehend beschriebenen Schneidkörper, die Vorschubeinrichtung zumindest teilweise umgibt. Günstigerweise ist dann der Schneidkörper koaxial zur Vorschubeinrichtung ausgerichtet.

Von Vorteil ist es, wenn die Vorschubeinrichtung drehbeweglich zur Schneideinrichtung ausgebildet ist. Wird die Schneideinrichtung wie vorstehend beschrieben zur Erzeugung einer in sich oder im Wesentlichen in sich geschlossenen Schnittlinie am Knochen drehend angetrieben, lässt sich dadurch beispielsweise die Trägheit der Aufsatzvorrichtung verringern, da es nicht erforderlich ist, die Vorschubeinrichtung ebenfalls anzutreiben. Die Vorschubeinrichtung kann, etwa mittels des vorstehend beschriebenen mindestens einen Eingriffselementes des mindestens einen Vorschubgliedes, in den Knochen eingreifen und relativ zu diesem verdrehgesichert sein, während die Schneideinrichtung eine Drehbewegung relativ zur Vorschubeinrichtung und zum Knochen ausführt.

Vorzugsweise ist das mindestens eine Vorschubglied verdrehgesichert zur Vorschubeinrichtung ausgebildet, das heißt zum Rest der Vorschubeinrichtung, welche das mindestens eine Vorschubglied umfasst. Auf diese Weise lässt sich ein Verschleiß der Vorschubeinrichtung vermeiden, der dann entstehen könnte, wenn das mindestens eine Vorschubglied drehbeweglich zur Vorschubeinrichtung ausgebildet wäre. Bei der zuletzt beschriebenen Ausführungsform wird dadurch auch die Möglichkeit gegeben, die Vorschubeinrichtung als Ganzes, das heißt einschließlich des mindestens einen Vorschubgliedes, drehbeweglich zur Schneideinrichtung auszubilden.

Als günstig hat es sich erwiesen, wenn die Vorschubeinrichtung relativ zur mindestens einen Schneidkante linear beweglich ist. Dies erlaubt es, die Vorschubeinrichtung als Ganzes relativ zur mindestens einen Schneidkante linear zu bewegen und auf diese Weise den Abstand zwischen dem mindestens einen Vorschubglied und der mindestens einen Schneidkante zu variieren. Unabhängig davon kann vorgesehen sein, dass das mindestens eine Vorschubglied zum Rest der Vorschubeinrichtung linear beweglich ausgebildet ist.

Bevorzugt definiert die Aufsatzvorrichtung eine Achse, entlang derer die Vorschubeinrichtung relativ zur mindestens einen Schneidkante linear beweglich ausgebildet ist. Dadurch lässt sich ein zuverlässiger Betrieb der Aufsatzvorrichtung sicherstellen. Die Achse ist vorteilhafterweise identisch mit einer Achse, entlang derer das mindestens eine Vorschubglied relativ zur mindestens einen Schneidkante beweglich ausgebildet ist.

Vorzugsweise bildet die Schneideinrichtung mit der Vorschubeinrichtung eine Schraubverbindung aus. Mittels der Schraubverbindung ist es möglich, die Schneideinrichtung und die Vorschubeinrichtung sowohl drehbeweglich zueinander als auch linear beweglich zueinander auszubilden, wobei die Linearbewegung von der Drehbewegung entkoppelt werden kann. Mittels der Schraubverbindung kann ferner insbesondere eine Schraubführung definiert werden, zur Führung sowohl der Linearbewegung als auch der Drehbewegung der Vorschubeinrichtung und der Schneideinrichtung relativ zueinander.

Zur konstruktiv einfachen Ausbildung der Schraubverbindung hat es sich als günstig erwiesen, wenn die Schneideinrichtung mit der Vorschubeinrichtung in Eingriff steht. Insbesondere kann vorgesehen sein, dass die Vorschubeinrichtung ein Außengewinde aufweist, welches mit einem Innengewinde der Schneideinrichtung zusammenwirkt zur Ausbildung der Schraubverbindung und zur Definition der Schraubführung.

Vorteilhafterweise ist die Schneideinrichtung von einer Betriebsstellung, in der sie mit der Vorschubeinrichtung in Eingriff steht, in eine Lösestellung überführbar, in der der Eingriff zwischen der Schneideinrichtung und der Vorschubeinrichtung aufgehoben ist und umgekehrt. Dies erleichtert die Handhabung der Aufsatzvorrichtung. Beispielsweise kann der Eingriff zwischen der Schneideinrichtung und der Vorschubeinrichtung zum Versetzen der Vorschubeinrichtung relativ zur Schneideinrichtung aufgehoben werden, indem die Schneideinrichtung in die Lösestellung überführt wird. Nach dem Versetzen der Vorschubeinrichtung relativ zur Schneideinrichtung kann die Schneideinrichtung wieder in die Betriebsstellung überführt werden.

Vorzugsweise weist die Aufsatzvorrichtung eine Rückstelleinrichtung auf, mit der die Schneideinrichtung von der Lösestellung in die Betriebsstellung überführbar ist. Dies bewirkt eine weitere Erleichterung der Handhabung der Aufsatzvorrichtung. Es kann insbesondere vorgesehen sein, dass das Überführen der Schneideinrichtung von der Lösestellung in die Betriebsstellung ohne Zutun eines Benutzers erfolgt.

Eine konstruktiv einfache Ausgestaltung der zuletzt genannten Ausführungsform kann dadurch erzielt werden, dass die Rückstelleinrichtung ein elastisches Element umfasst, entgegen dessen Vorspannung die Schneideinrichtung in der Betriebsstellung mit der Vorschubeinrichtung in Eingriff steht. Bei dem elastischen Element kann es sich zum Beispiel um Federn jedweder Art handeln wie etwa Spiralfedern, Blattfedern, Gasdruckfedern, Gummimanschetten oder dergleichen.

Besonders einfach ist die Aufsatzvorrichtung handhabbar, wenn die Schneideinrichtung so ausgebildet ist, dass sie manuell von der Betriebsstellung in die Lösestellung und/oder umgekehrt überführbar ist. Die Schneideinrichtung kann zum Beispiel eine Betätigungseinrichtung umfassen, durch dessen Betätigung ein Benutzer die Schneideinrichtung von der Lösestellung in die Betriebsstellung und/oder umgekehrt überführen kann.

Eine konstruktiv einfache Ausgestaltung und kompakte Bauform der Aufsatzvorrichtung kann dadurch erzielt werden, dass die Vorschubeinrichtung einen das mindestens eine Vorschubglied zumindest teilweise umgebenden hülsenartigen Vorschubkörper aufweist, der mit der Schneideinrichtung in Eingriff steht. Der Vorschubkörper kann insbesondere mit der Schneideinrichtung über ein Gewinde in Eingriff stehen und mit dieser eine Schraubverbindung ausbilden. Darüber hinaus kann der Vorschubkörper eine Führung für das mindestens eine Vorschubglied für dessen Bewegung relativ zur mindestens einen Schneidkante definieren.

Eine noch kompaktere Bauform der Aufsatzvorrichtung kann dadurch erzielt werden, dass der Vorschubkörper die Kraftbeaufschlagungseinrichtung zumindest teilweise umgibt.

Günstig ist es, wenn der Vorschubkörper mindestens einen dem mindestens einen Vorschubglied zur Begrenzung dessen Bewegung relativ zur mindestens einen Schneidkante zugeordneten Anschlag umfasst oder ausbildet. Vorzugsweise umfasst oder bildet der Vorschubkörper zwei Anschläge aus, die die Bewegung des mindestens einen Vorschubgliedes sowohl in distaler als auch in proximaler Richtung begrenzen.

Eine konstruktiv einfache Bauform und ein zuverlässiger Betrieb der Aufsatzvorrichtung lassen sich dadurch erzielen, dass die Abstützeinrichtung einen zumindest teilweise hülsenartigen Abstützkörper aufweist und dass das mindestens eine Abstützglied an einer dessen Enden angeordnet ist.

Für einen zuverlässigen Betrieb und eine kompakte Bauform der Aufsatzvorrichtung hat es sich als vorteilhaft erwiesen, wenn die Abstützeinrichtung koaxial zur Schneideinrichtung und/oder koaxial zur Vorschubeinrichtung ausgerichtet ist. Besonders bevorzugt ist die Abstützeinrichtung sowohl zur Schneideinrichtung als auch zur Vorschubeinrichtung koaxial ausgerichtet.

Vorteilhafterweise umgibt die Abstützeinrichtung die Schneideinrichtung und/oder die Vorschubeinrichtung zumindest teilweise. Besonders vorteilhaft ist es, wenn die Abstützeinrichtung die Schneideinrichtung und die Vorschubeinrichtung vollständig umgibt. Dies erlaubt es, die Aufsatzvorrichtung an einem Bereich des zu durchschneidenden Knochens abzustützen, der außerhalb der Schnittlinie angeordnet ist, so dass die Aufsatzvorrichtung am Knochen wirkungsvoll gegen Eindringen in das Körperinnere des Patienten abgestützt werden kann. Insbesondere kann die Abstützeinrichtung mittels des vorstehend beschriebenen Abstützkörpers am Knochen abgestützt werden, der bevorzugt koaxial zur Schneideinrichtung und zur Vorschubeinrichtung ausgerichtet ist.

Günstig ist, wenn die Abstützeinrichtung drehbeweglich zur Schneideinrichtung ausgebildet ist. Wie oben erläutert, lässt sich durch eine Drehbewegung eine geschlossene oder im Wesentlichen geschlossene Schnittlinie am Knochen erzeugen. Ist die Abstützeinrichtung drehbeweglich zur Schneideinrichtung, kann sie sich somit beispielsweise verdrehgesichert relativ zum Knochen an diesem abstützen, wobei das mindestens eine Abstützglied mit dem Knochen in Eingriff steht.

Vorteilhafterweise ist die Abstützeinrichtung relativ zur mindestens einen Schneidkante linear beweglich ausgebildet. Dies erlaubt es, die Abstützeinrichtung als Ganzes relativ zur mindestens einen Schneidkante zu bewegen. Während des Durchschneidens des Knochens, bei dem sich die Aufsatzvorrichtung mit der Abstützeinrichtung am Knochen abstützt, kann das mindestens eine Schneidelement in den Knochen eindringen.

Ein zuverlässiger Betrieb der Aufsatzvorrichtung kann dadurch sichergestellt werden, dass die Abstützeinrichtung entlang einer von der Aufsatzvorrichtung definierten Achse relativ zur mindestens einen Schneidkante linear beweglich ausgebildet ist.

Vorzugsweise bildet die Abstützeinrichtung mit der Schneideinrichtung eine Schraubverbindung aus. Dadurch lässt sich sicherstellen, dass die Abstützeinrichtung relativ zur Schneideinrichtung sowohl linear beweglich als auch drehbeweglich ausgebildet ist, wobei die Linearbewegung von der Drehbewegung entkoppelt werden kann. Insbesondere kann über die Schraubverbindung ferner eine Schraubführung definiert werden, zur Führung sowohl der Linearbewegung als auch der Drehbewegung der Abstützeinrichtung und der Schneideinrichtung relativ zueinander.

Auf konstruktiv einfache Weise kann die Schraubvorrichtung ausgebildet werden, wenn die Abstützeinrichtung mit der Schneideinrichtung in Eingriff steht, insbesondere über zusammenwirkende Gewinde. Beispielsweise kann die Abstützeinrichtung ein Innengewinde aufweisen, das mit einem Außengewinde der Schneideinrichtung zur Ausbildung der Schraubverbindung zusammenwirkt.

Günstigerweise weist die Aufsatzvorrichtung eine der Schneideinrichtung zugeordnete erste Kupplungseinrichtung auf mit einem mit dem mindestens einen Schneidelement in Wirkverbindung stehenden ersten Kupplungsglied und einem antreibbaren zweiten Kupplungsglied, das mit dem ersten Kupplungsglied zusammenwirkt. Über die erste Kupplungseinrichtung kann eine Antriebskraft auf das mindestens eine Schneidelement übertragen werden, wobei die Antriebskraft von einer Antriebseinrichtung ausgehen kann, mit der die Aufsatzvorrichtung koppelbar ist. Beispielsweise kann die eingangs genannte chirurgische Vorrichtung zum Durchschneiden eines Knochens eine derartige Antriebseinrichtung aufweisen. Das erste Kupplungsglied sowie das zweite Kupplungsglied können an der Schneideinrichtung angeordnet oder von dieser umfasst werden, es ist allerdings auch möglich, dass sie von einer mit der Schneideinrichtung koppelbaren Getriebeeinrichtung umfasst werden oder daran angeordnet sind.

Vorzugsweise ist das erste Kupplungsglied mit dem zweiten Kupplungsglied durch Kraftbeaufschlagung des mindestens einen Schneidelementes und/oder des mindestens einen Vorschubgliedes in proximaler Richtung in Wirkverbindung bringbar. Dies gibt die Möglichkeit, das erste und das zweite Kupplungsglied dadurch miteinander in Wirkverbindung zu bringen, dass die Aufsatzvorrichtung auf den zu durchschneidenden Knochen aufgesetzt wird. Übt der Operateur dabei eine in distaler Richtung wirkende Kraft auf den Knochen aus, kann dessen Gegenkraft dazu verwendet werden, das erste und zweite Kupplungsglied miteinander in Wirkverbindung zu bringen. Dies begünstigt die Handhabbarkeit der Aufsatzvorrichtung.

Von Vorteil ist es, wenn die Aufsatzvorrichtung eine Rückstelleinrichtung umfasst, entgegen deren Wirkung das erste Kupplungsglied mit dem zweiten Kupplungsglied in Wirkverbindung bringbar ist. Durch die Rückstelleinrichtung lässt sich sicherstellen, dass das erste und das zweite Kupplungsglied nicht dauerhaft miteinander in Wirkverbindung stehen, so dass das mindestens eine Schneidelement selbst bei angetriebenem erstem Kupplungsglied nicht dauerhaft angetrieben wird. Dadurch ist die Aufsatzvorrichtung auf sicherere Weise handhabbar.

Bevorzugt ist die Aufsatzvorrichtung so ausgebildet, dass das erste Kupplungsglied mit einer vom zweiten Kupplungsglied wegweisenden Kraft beaufschlagbar ist, zumindest sofern der Abstand des mindestens einen Vorschubgliedes von der mindestens einen Schneidkante einen Mindestabstand überschreitet. Wie vorstehend erläutert, kann das Überschreiten eines Mindestabstandes ein Indiz dafür sein, dass der Knochen mittels der Schneideinrichtung durchgeschnitten worden ist. Bei dieser Ausführungsform wird daher ermöglicht, dass die Wirkverbindung zwischen dem ersten Kupplungsglied und dem zweiten Kupplungsglied nach dem Durchschneiden des Knochens aufgehoben wird. Die Schneideinrichtung wird auf diese Weise nach dem Durchschneiden des Knochens automatisch von einer gegebenenfalls vorhandenen Antriebseinrichtung entkoppelt wird. Dadurch lässt sich die Verletzungsgefahr des Patienten minimieren und die Handhabung der Aufsatzvorrichtung erleichtern. Zur Kraftbeaufschlagung kann beispielsweise die vorstehend beschriebene Rückstelleinrichtung eingesetzt werden, die etwa in Form eines zwischen dem ersten Kupplungsglied und dem zweiten Kupplungsglied angeordneten elastischen Elementes ausgebildet sein kann.

Bei einer konstruktiv einfachen Ausgestaltung der Aufsatzvorrichtung sind das erste Kupplungsglied und das zweite Kupplungsglied als ineinandergreifende Vorsprünge ausgebildet.

Vorteilhafterweise weist die Aufsatzvorrichtung eine der Abstützeinrichtung zugeordnete zweite Kupplungseinrichtung auf mit einem mit dem mindestens einen Abstützglied in Wirkverbindung stehenden dritten Kupplungsglied und einem antreibbaren vierten Kupplungsglied, das mit dem dritten Kupplungsglied zusammenwirkt. Wie im Fall der vorstehend beschriebenen ersten Kupplungseinrichtung ist es dadurch möglich, eine Antriebskraft auf die Abstützeinrichtung nur dann zu übertragen, wenn das dritte Kupplungsglied mit dem vierten Kupplungsglied in Wirkverbindung steht. Es kann vorgesehen sein, dass das dritte Kupplungsglied mit dem Abstützglied nur mittelbar in Wirkverbindung steht. Zum Beispiel kann das dritte Kupplungsglied an der Schneideinrichtung angeordnet sein, welche mit der Abstützeinrichtung eine Schraubverbindung ausbildet. Ein Antreiben des dritten Kupplungsgliedes kann dann eingesetzt werden, um wie vorstehend erläutert, eine Drehbewegung der Schneideinrichtung relativ zur Abstützeinrichtung zu bewirken.

Bevorzugt ist das dritte Kupplungsglied mit dem vierten Kupplungsglied durch Kraftbeaufschlagung des mindestens einen Abstützgliedes in proximaler Richtung in Wirkverbindung bringbar. Dies kann beispielsweise dadurch erfolgen, dass die Aufsatzvorrichtung vom Operateur mit einer in distaler Richtung wirkenden Kraft auf den zu durchschneidenden Knochen aufgesetzt wird, durch dessen Gegenkraft in proximaler Richtung das dritte Kupplungsglied mit dem vierten Kupplungsglied in Wirkverbindung bringbar ist. Dies erleichtert die Handhabung der Aufsatzvorrichtung.

Von Vorteil ist es, wenn die Aufsatzvorrichtung eine Rückstelleinrichtung umfasst, entgegen deren Wirkung das dritte Kupplungsglied mit dem vierten Kupplungsglied in Wirkverbindung bringbar ist. Dadurch ist sichergestellt, dass das dritte Kupplungsglied und das vierte Kupplungsglied nicht dauerhaft miteinander in Wirkverbindung stehen.

Besonders günstig ist es, wenn das vierte Kupplungsglied mit der ersten Kupplungseinrichtung mechanisch gekoppelt ist. Dies erlaubt es, das vierte Kupplungsglied nur dann anzutreiben, wenn das erste Kupplungsglied und das zweite Kupplungsglied miteinander in Wirkverbindung stehen.

Auf konstruktiv einfache Weise lässt sich die zweite Kupplungseinrichtung ausbilden, wenn das dritte Kupplungsglied und das vierte Kupplungsglied als ineinandergreifende Vorsprünge ausgebildet sind.

Vorzugsweise bilden die Schneideinrichtung und die Vorschubeinrichtung zusammen zumindest teilweise eine vormontierte und austauschbare Baueinheit. Es kann sich daher um eine Baueinheit der Aufsatzvorrichtung oder auch um eine Baueinheit der nachfolgend noch erläuterten erfindungsgemäßen Vorrichtung zum Durchschneiden eines Knochens handeln. Dies verbessert die Vielseitigkeit der Aufsatzvorrichtung beziehungsweise der chirurgischen Vorrichtung und begünstig überdies eine kompakte Bauform derselben. Beispielsweise kann vorgesehen sein, dass zur Erzeugung von Öffnungen verschiedenen Durchmessers am zu durchschneidenden Knochen unterschiedliche Baueinheiten vorgesehen sind, die jeweils eine Schneideinrichtung mit daran angepasster Vorschubeinrichtung umfassen.

Um die Aufsatzvorrichtung antreiben zu können, ist es von Vorteil, wenn die Aufsatzvorrichtung eine Anschlusseinrichtung zum Anschluss der Aufsatzvorrichtung an eine Antriebseinrichtung aufweist.

Wie bereits erwähnt, betrifft die Erfindung auch eine chirurgische Vorrichtung zum Durchschneiden eines Knochens mit mindestens einer Aufsatzvorrichtung.

Bei einer gattungsgemäßen chirurgischen Vorrichtung wird die eingangs gestellte Aufgabe erfindungsgemäß dadurch gelöst, dass die chirurgische Vorrichtung mindestens eine erfindungsgemäße Aufsatzvorrichtung umfasst.

Die erfindungsgemäße chirurgische Vorrichtung weist damit die bereits im Zusammenhang mit der Erläuterung der erfindungsgemäßen Aufsatzvorrichtung beschriebenen Vorteile auf.

Insbesondere ist es von Vorteil, wenn die mindestens eine Aufsatzvorrichtung der erfindungsgemäßen chirurgischen Vorrichtung als eine der vorstehend beschriebenen Aufsatzvorrichtungen ausgebildet ist. Die chirurgische Vorrichtung weist dann die bei der Erläuterung dieser Aufsatzvorrichtungen erwähnten weiteren Vorteile auf.

Wie bereits erläutert, weist die chirurgische Vorrichtung günstigerweise eine mit der Schneideinrichtung koppelbare Antriebseinrichtung zum Antreiben der Schneideinrichtung auf. Zur Kopplung der Antriebseinrichtung mit der Schneideinrichtung umfasst die chirurgische Vorrichtung vorzugsweise eine zu einer Anschlusseinrichtung der Schneideinrichtung korrespondierend ausgebildete Anschlusseinrichtung. Alternativ oder zusätzlich kann die chirurgische Vorrichtung so ausgebildet sein, dass die Schneideinrichtung manuell antreibbar ist. Die Antriebseinrichtung ist vorzugsweise als elektrische Antriebseinrichtung ausgebildet, es kann sich hierbei aber auch um eine pneumatische, hydraulische, unter Einsatz eines Brennstoffes arbeitende oder auch andersartig ausgebildete Antriebseinrichtung handeln.

Bevorzugt ist die Antriebseinrichtung so ausgebildet, dass die Schneideinrichtung rotierend antreibbar ist. Unter "rotierend antreibbar" wird vorliegend verstanden, dass ein auf der mindestens einen Schneidkante angeordneter Bezugspunkt sich entlang einer Kreislinie bewegt, vorteilhafterweise zur Erzeugung einer in sich geschlossenen Schnittlinie am Knochen.

Es kann auch vorgesehen sein, dass die Antriebseinrichtung so ausgebildet ist, dass die Schneideinrichtung oszillierend antreibbar ist. In diesem Fall führt ein auf der mindestens einen Schneidkante angeordneter Bezugspunkt eine oszillierende Bewegung aus. Dies kann, je nach Ausbildung der Schneideinrichtung, eine in sich geschlossene, im Wesentlichen in sich geschlossene oder nicht in sich geschlossene Schnittlinie am Knochen zur Folge haben.

Darüber hinaus kann vorgesehen sein, dass die Antriebseinrichtung so ausgebildet ist, dass die Schneideinrichtung gemäß einem Pilgerschrittverfahren antreibbar ist. Ein auf der Schneidkante angeordneter Bezugspunkt führt dadurch eine fortschreitende Vorwärts-/Rückwärtsbewegung aus, die beispielsweise dadurch zustande kommt, dass einer rotierenden Bewegung eine oszillierenden Bewegung überlagert wird.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung, teilweise in Schnittansicht, einer ersten Ausführungsform einer Aufsatzvorrichtung;
- Figur 2:: eine weitere perspektivische Darstellung der Aufsatzvorrichtung aus Figur 1;
- Figur 3:: eine Schnittansicht längs der Linie 3-3 in Figur 1 vor dem Durchschneiden eines Schädelknochens;
- Figur 4:: die Aufsatzvorrichtung und den Schädelknochen aus Figur 3 während des Schneidvorganges;
- Figur 5:: die Aufsatzvorrichtung und den Schädelknochen aus Figur 3 nach dem Schneidvorgang;
- Figur 6:: eine Teilschnittansicht einer zweiten Ausführungsform einer Aufsatzvorrichtung mit einer eine Betriebsstellung einnehmenden Schneidvorrichtung;
- Figur 7:: die Aufsatzvorrichtung aus Figur 6, deren Schneideinrichtung von der Betriebsstellung in eine Lösestellung überführt wurde;
- Figur 8:: eine Teilschnittansicht einher dritten Ausführungsform, die eine bevorzugte Ausführungsform einer erfindungsgemäßen Aufsatzvorrichtung vor dem Durchschneiden eines Schädelknochens darstellt;
- Figur 9:: die Aufsatzvorrichtung und den Schädelknochen aus Figur 8 während des Schneidvorganges;
- Figur 10:: die Aufsatzvorrichtung und den Schädelknochen aus Figur 8 nach dem Schneidvorgang;
- Figur 11:: eine Teilschnittansicht einher vierten Ausführungsform, die eine weitere bevorzugte Ausführungsform einer erfindungsgemäßen Aufsatzvorrichtung vor dem Durchschneiden eines Schädelknochens darstellt;
- Figur 12:: eine vergrößerte Darstellung von Detail A in Figur 11;
- Figur 13:: die Aufsatzvorrichtung und den Schädelknochen aus Figur 11 zu Beginn des Schneidvorganges;
- Figur 14:: eine vergrößerte Darstellung von Detail B in Figur 13;
- Figur 15:: die Aufsatzvorrichtung und den Schädelknochen aus Figur 11 nach dem Schneidvorgang;
- Figur 16:: eine vergrößerte Darstellung von Detail C in Figur 15;
- Figur 17:: eine erste Ausführungsform einer chirurgischen Vorrichtung zum Durchschneiden eines Knochens, umfassend die Aufsatzvorrichtung aus Figur 1;
- Figur 18:: eine zweite Ausführungsform einer chirurgischen Vorrichtung, umfassend die Aufsatzvorrichtung aus Figur 6;
- Figur 19:: eine dritte, bevorzugte Ausführungsform einer erfindungsgemäßen chirurgischen Vorrichtung, umfassend die Aufsatzvorrichtung aus Figur 8 und
- Figur 20:: eine vierte, bevorzugte Ausführungsform einer erfindungsgemäßen chirurgischen Vorrichtung, umfassend die Aufsatzvorrichtung aus Figur 11.

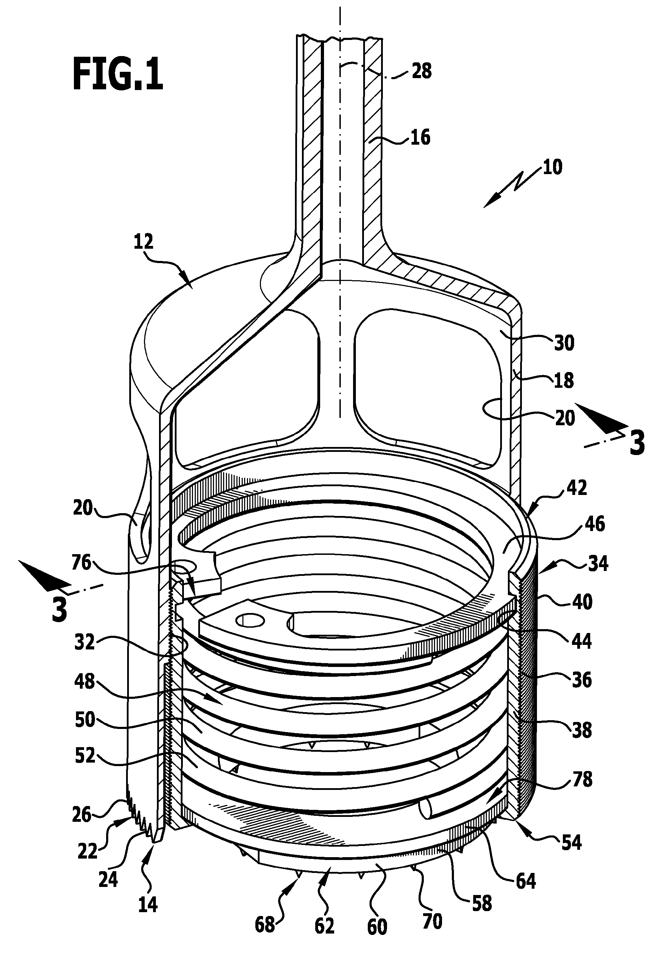

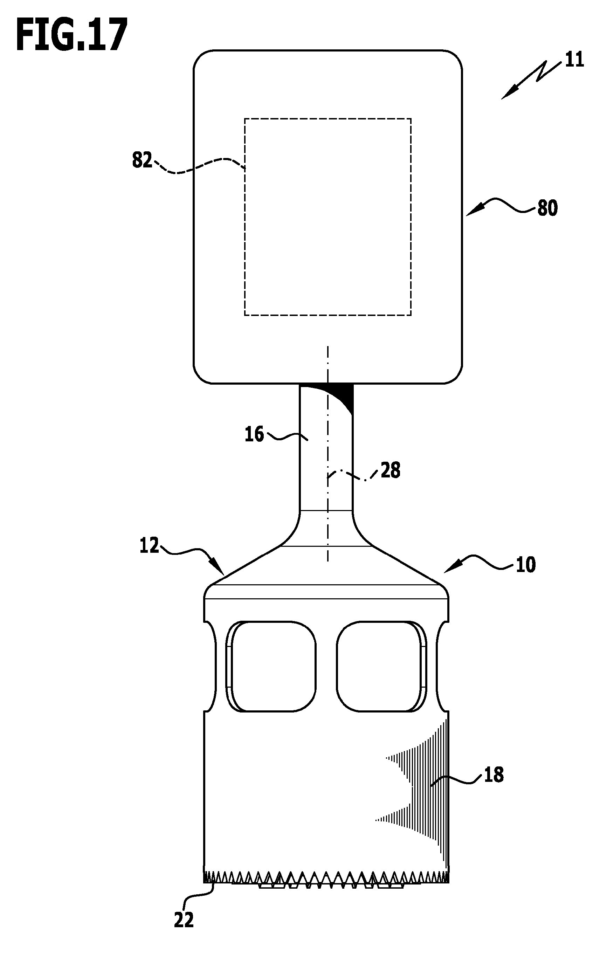

Eine erste Ausführungsform einer Aufsatzvorrichtung ist in den Figuren 1 bis 5 dargestellt und dort insgesamt mit dem Bezugszeichen 10 belegt. Die Aufsatzvorrichtung 10 kommt als Zubehör oder Ergänzungsvorrichtung für eine chirurgische Vorrichtung zum Durchschneiden eines Knochens, insbesondere des menschlichen Schädels, zum Einsatz oder bildet einen Bestandteil derselben, insbesondere eine Baueinheit. Eine erste Ausführungsform einer chirurgischen Vorrichtung zum Durchschneiden eines Knochens ist in der Figur 17 dargestellt, dort mit dem Bezugszeichen 11 belegt und wird weiter unten erläutert.

Die Aufsatzvorrichtung 10 umfasst eine Schneideinrichtung 12, die ein erstes in der Zeichnung nicht dargestelltes proximales Ende und ein zweites in der Zeichnung dargestelltes distales Ende 14 aufweist. "Proximal" und "distal" ist vorliegend nicht nur für die Aufsatzvorrichtung 10, sondern auch für die weiteren nachfolgend beschriebenen Aufsatzvorrichtungen 100, 200 und 300 dahingehend zu verstehen, dass "proximal" und "distal" auf den die Aufsatzvorrichtung 10 benutzenden Operateur aufzufassen sind. Dies bedeutet, dass das proximale Ende der Aufsatzvorrichtung 10 dem Operateur und insbesondere dessen Händen, mit denen er die Aufsatzvorrichtung 10 üblicherweise führt, näher gelegen ist als das distale Ende 14 der Aufsatzvorrichtung 10. In der Zeichnung ist das distale Ende 14 der Aufsatzvorrichtung 10 jeweils unten dargestellt, und das nicht dargestellte proximale Ende der Aufsatzvorrichtung 10 läge dementsprechend jeweils oben in den jeweiligen Figuren. Dies gilt in gleicher Weise für die nachfolgend beschriebenen weiteren Ausführungsformen 100, 200 und 300 der Aufsatzvorrichtung.

Ausgehend vom proximalen Ende weist die Schneideinrichtung 12 einen Schaft 16 auf, der sich in distaler Richtung trichterförmig erweitert und in einen hülsenartig ausgebildeten Schneidkörper 18 übergeht. Der Schneidkörper 18 ist insbesondere hohlzylindrisch ausgebildet und umfasst zur Gewichtsreduktion eine Mehrzahl von Durchbrechungen 20. An seinem distalen Ende weist der Schneidkörper 18 eine Vielzahl von Schneidelementen 22 auf, die von dem vom Schneidkörper 18 definierten Zylindermantel in distaler Richtung abstehen. Die Schneidelemente 22 sind in Form scharfer Zähne 24 ausgebildet, die gemeinsam eine kreisförmige Schneidkante 26 der Schneideinrichtung 12 bilden. Die Schneideinrichtung 12 ist insgesamt rotationssymmetrisch bezüglich einer Achse 28 ausgestaltet, die eine Achse der Aufsatzvorrichtung 10 definiert.

An einer Innenseite 30 umfasst der Schneidkörper 18 distal zu den Durchbrechungen 20 ein Innengewinde 32, über welches die Schneideinrichtung 12 mit einer Vorschubeinrichtung 34 der Aufsatzvorrichtung 10 in Eingriff steht, die hierfür ein Außengewinde 36 aufweist. Die Vorschubeinrichtung 34 ist vom Schneidkörper 18 umgeben und koaxial zur Schneideinrichtung 12 ausgerichtet. Sie umfasst einen hülsenartigen und in erster Näherung hohlzylindrisch ausgebildeten Vorschubkörper 38, an dessen Außenseite 40 das Außengewinde 36 angeordnet ist.

Nahe seinem proximalen Ende 42 weist der Vorschubkörper 38 innenseitig eine Ringnut 44 auf, in welcher ein Federring 46 eingespannt ist, an welchem eine vom Vorschubkörper 38 umgebene Kraftbeaufschlagungseinrichtung 48 in Form eines als Spiralfeder 50 ausgestalteten elastischen Elementes 52 anliegt.

Am distalen Ende 54 des Vorschubkörpers 38 ist die zylindrische Wand des Vorschubkörpers 38 in Richtung der Achse 28 nach innen gebogen und bildet so einen Ringflansch 56. Dieser wird innenseitig von einem näherungsweise hohlzylindrischen Abschnitt 58 eines im Folgenden als Stempel 60 bezeichneten Vorschubgliedes 62 der Vorschubeinrichtung 34 durchgriffen. An den hohlzylindrischen Abschnitt 58 schließt sich in proximaler Richtung ein Ringabschnitt 64 des Stempels 60 an.

Der Ringabschnitt 64 liegt am distalen Ende der Spiralfeder 50 an, die somit zwischen dem Federring 46 und dem Stempel 60 eingespannt und insbesondere vorgespannt ist, um den Stempel 60 mit einer Kraft in distaler Richtung zu beaufschlagen. Durch Anliegen des Ringsabschnittes 64 des Stempels 60 an dem Ringflansch 56 des Vorschubkörpers 38 kann verhindert werden, dass der Stempel 60 durch die Spiralfeder 50 in distaler Richtung aus dem Vorschubkörper 38 herausgedrückt wird.

Am distalen Ende umfasst der Stempel 60 eine näherungsweise ringförmige Anlagefläche 66, aus der in distaler Richtung eine Mehrzahl von Eingriffselementen 68 in Form von scharfkantigen Leisten 70 hervorspringen.

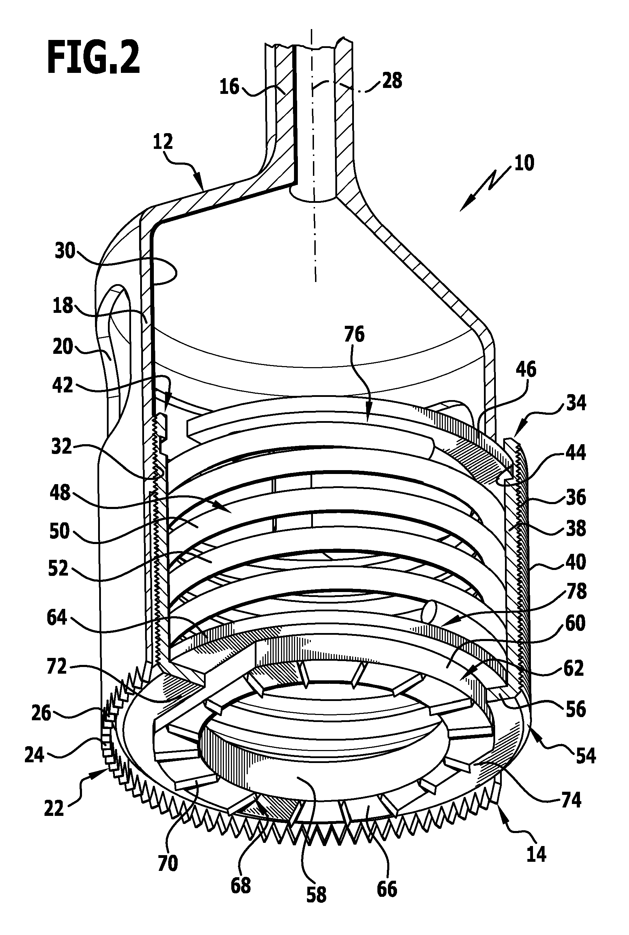

Relativ zum Vorschubkörper 38 ist der Stempel 60 längs der Achse 28 beweglich ausgebildet, wobei der Vorschubkörper 38 einen Führungskörper für den Stempel 60 definiert. Zur Führung des Stempels 60 kann dessen Ringabschnitt 64 innenseitig an Vorschubkörper 38 anliegen und dessen hohlzylindrischer Abschnitt 58 innenseitig am Ringflansch 56. Dabei sind vom hohlzylindrischen Abschnitt 58 derart randseitig zwei Zylindersegmente entfernt und weist der Ringflansch 56 innenseitig derart zwei Ringsegmente 72 und 74 auf (Figur 2), dass der Stempel 60 insgesamt verdrehgesichert zum Rest der Vorschubeinrichtung 34 ausgestaltet ist.

Der Bewegungsumfang des Stempels 60 relativ zum Vorschubkörper 38 wird durch zwei Anschläge 76 sowie 78 begrenzt. Der proximale Anschlag 76 wird durch den Federring 46 ausgebildet, an dem die Spiralfeder 50 anliegt und über diese auch mittelbar der Stempel 60. Der distale Anschlag 78 wird durch den Ringflansch 56 gebildet, an dem, wie bereits erwähnt, der Ringabschnitt 64 des Stempels 60 anliegen kann, sofern der Stempel 60 nicht durch Kraftbeaufschlagung in proximaler Richtung von diesem weg bewegt wird.

Durch seine Beweglichkeit relativ zum Vorschubkörper 38 ist der Stempel 60 somit auch relativ zu den Zähnen 24 und der durch sie definierten Schneidkante 26 beweglich ausgebildet, und er kann relativ zur Schneidkante 26 einen veränderlichen Abstand einnehmen.

Infolge des bestehenden Eingriffes des Innengewindes 32 der Schneideinrichtung 12 und des Außengewindes 36 der Vorschubeinrichtung 34 bilden die Schneideinrichtung 12 und die Vorschubeinrichtung 34 miteinander eine Schraubverbindung aus, durch die insbesondere eine Schraubführung definiert wird zur Führung einer Relativbewegung der Schneideinrichtung 12 und der Vorschubeinrichtung 34. Zum einen wird durch die Schraubverbindung eine Drehbewegung der Schneideinrichtung 12 relativ zur Vorschubeinrichtung 34 ermöglicht. Darüber hinaus ist es auch möglich, die Vorschubeinrichtung 34 relativ zur Schneideinrichtung 12 linear zu bewegen, und zwar entlang der Achse 28 sowohl in distaler als auch in proximaler Richtung.

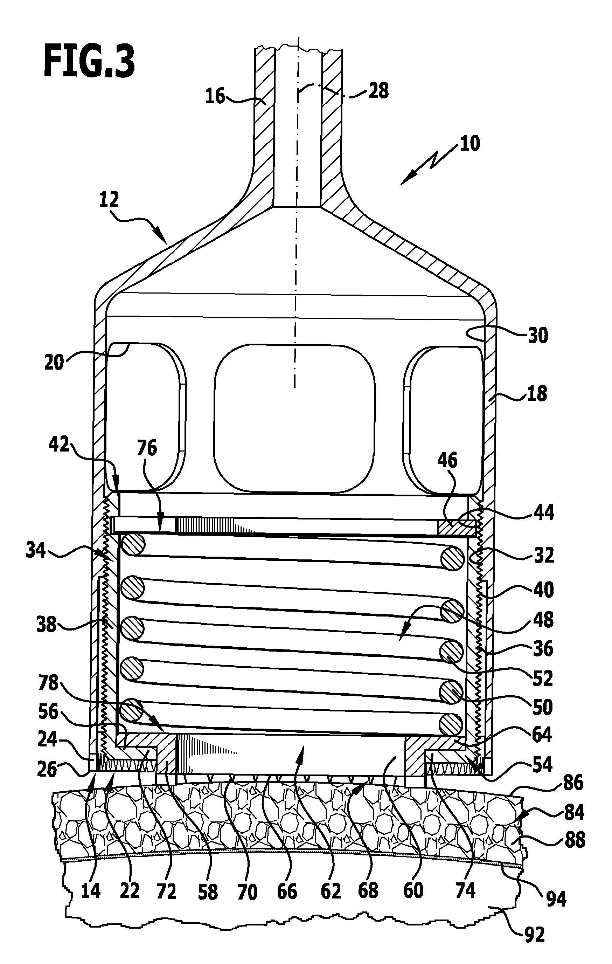

Wie nachfolgend insbesondere unter Bezugnahme auf die Figuren 3 bis 5 beschrieben wird, ist zum Durchschneiden eines Knochens mittels der Schneideinrichtung 12 folgendermaßen vorzugehen:

Zunächst ist die Schneideinrichtung 12 mit einer an sich bekannten, in der Zeichnung nicht gezeigten und an ihrem proximalen Ende angeordneten Anschlusseinrichtung mit einer Antriebseinheit 80 (Figur 17) zu verbinden. Die Antriebseinheit 80 weist hierfür eine zur Anschlusseinheit der Schneideinrichtung 12 korrespondierend ausgebildete Anschlusseinrichtung auf, welche in der Zeichnung ebenfalls nicht zu sehen ist und mit der Anschlusseinrichtung der Schneideinrichtung 12 auf an sich bekannte und deswegen nicht näher erläuterte Weise verbunden werden kann. Zusammen mit der Antriebseinheit 80 bildet die Schneideinrichtung 12 die bereits erwähnte bevorzugte Ausführungsform einer chirurgischen Vorrichtung 11 zum Durchschneiden eines Knochens.

Zum Antreiben der Schneideinrichtung 12 umfasst die Antriebseinheit 80 eine mit ihrer Anschlusseinrichtung gekoppelte Antriebseinrichtung 82, die vorzugsweise als elektrische Antriebseinrichtung ausgebildet ist. Es kann sich hierbei aber auch um eine pneumatische, hydraulische, unter Einsatz eines Brennstoffes arbeitende oder auch andersartig ausgebildete Antriebseinrichtung handeln.

Die Antriebseinrichtung 82 ist so ausgebildet, dass die Schneideinrichtung 12 sowohl rotierend als auch oszillierend als auch gemäß einem Pilgerschrittverfahren antreibbar ist. Unter "rotierend antreibbar" wird vorliegend verstanden, dass ein Bezugspunkt auf der Schneidkante, beispielsweise einer der Zähne 24, eine vorzugsweise kontinuierliche Kreisbewegung ausführt, unter "oszillierend antreibbar" wird vorliegend verstanden, dass der Bezugspunkt eine periodische Hin- und Herbewegung ausführt, und unter "gemäß einem Pilgerschrittverfahren" wird vorliegend eine überlagerte rotierende und oszillierende Bewegung des Bezugspunktes verstanden, so dass dieser unter periodischer Vor- und Rückbewegung ebenfalls eine vorzugsweise kontinuierliche Kreisbewegung ausführt. Vereinfachend aber nicht beschränkend wird nachfolgend angenommen, dass die Schneideinrichtung 12 mittels der Antriebseinrichtung 82 rotierend angetrieben wird.

Zum Durchschneiden eines Knochens, beispielsweise des menschlichen Schädels, wie er in den Figuren 3 bis 5, 8 bis 11 sowie 13 und 15 abschnittsweise schematisch dargestellt und mit dem Bezugszeichen 84 belegt ist, ist die Schneideinrichtung 12 mit dem Stempel 60 voraus auf der Außenseite 86 des Schädelknochens 88 aufzusetzen (Figur 3). Durch Kraftbeaufschlagung der Schneideinrichtung 12 durch den Operateur in distaler Richtung wird ermöglicht, dass die Anlagefläche 66 des Stempels 60 an der Außenseite 86 des Schädelknochens 88 anliegt und die Leisten 70 in Eingriff mit dem Schädelknochen 88 geraten. Darüber hinaus wird der Stempel 60 relativ zum Vorschubkörper 38 und zur Schneidkante 26 in proximaler Richtung entgegen der Federkraft der Spiralfeder 50 verschoben, wobei der Federring 46 als proximaler Anschlag 76 für den Stempel 60 dient.

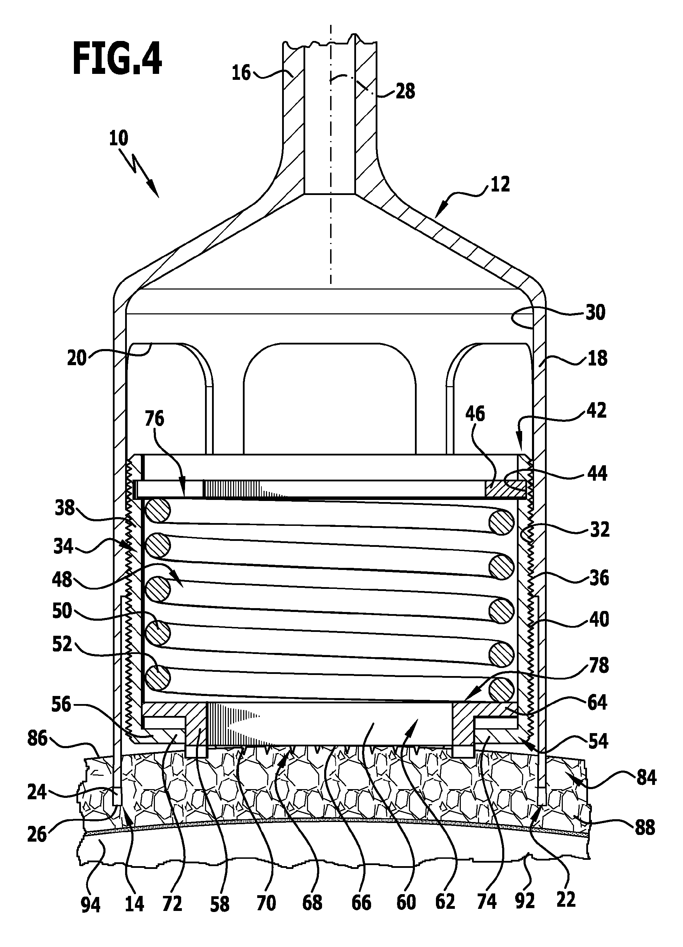

Durch bestehenden Eingriff zwischen dem Stempel 60 und dem Schädelknochen 88 ist der Stempel 60 und damit auch die gesamte Vorschubeinrichtung 34 relativ zum Schädelknochen 88 verdrehgesichert. Durch Aktivieren der Antriebseinrichtung 82 der Antriebseinheit 80 kann daraufhin die Schneideinrichtung 12 in eine Drehbewegung versetzt werden. Dies bewirkt, dass sich die Schneideinrichtung 12 relativ zur Vorschubeinrichtung 34 in distaler Richtung linear vorwärts bewegt, aufgrund der durch die Schneideinrichtung 12 und die Vorschubeinrichtung 34 definierten Schraubverbindung mittels des Innengewindes 32 der Schneideinrichtung 12 und des Außengewindes 36 der Vorschubeinrichtung 34. Durch fortdauernde Drehbewegung dringen die Zähne 24 und damit die durch sie definierte Schneidkante 26 in den Schädelknochen 88 ein und erzeugen dabei am Schädelknochen 88 eine kreisrunde in sich geschlossene und in der Zeichnung nicht dargestellte Schnittlinie (Figur 4).

Die Härte der Spiralfeder 50 ist derart bemessen, dass der Widerstand, den der Schädelknochen 88 der Schneideinrichtung 12 beim Durchschneiden entgegensetzt, durch den bestehenden Eingriff zwischen dem Stempel 60 und dem Schädelknochen 88 durch Kraftbeaufschlagung des Stempels 60 mittels der Spiralfeder 50 kompensiert werden kann. Damit kann die Drehbeweglichkeit der Vorschubeinrichtung 34 relativ zur Schneidbewegung 12 aufgrund der Verdrehsicherung der Vorschubeinrichtung 34 relativ zum Schädelknochen 88 sichergestellt werden.

Bei fortschreitendem Vortrieb der Schneidelemente 22 in den Schädelknochen 88 nimmt der Abstand des Stempels 60 relativ zur Schneidkante 26 aufgrund der Relativbewegung der Vorschubeinrichtung 34 und der Schneideinrichtung 12 zu. Ist der Schädelknochen 88 vollständig durchgeschnitten, entfällt die Gegenkraft des Schädelknochens 88, welche dieser der Kraftbeaufschlagung des Stempels 60 durch die Spiralfeder 50 entgegensetzt. Die Dicke des Schädelknochens 88 ist damit ein Maß für einen Mindestabstand, welchen der Stempel 60 relativ zur Schneidkante 26 einnehmen muss, damit die Gegenkraft des Schädelknochens 88 entfällt. Der Stempel 60 wirkt somit gewissermaßen als Detektionsglied für das Entfallen der Gegenkraft des Schädelknochens und damit als Detektionsglied dafür, wann der Schädelknochen 88 mittels der Schneideinrichtung 12 durchgeschnitten ist.

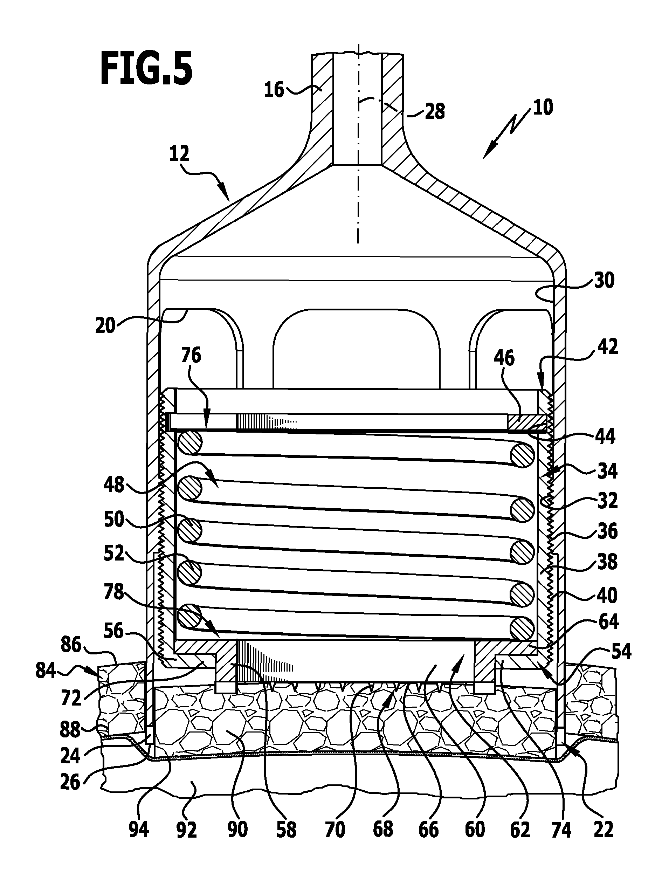

Ein von den Zähnen 24 eingefasstes ausgeschnittenes Knochensegment 90 wird nach dem Durchschneiden des Schädelknochens 88 aufgrund der Kraftbeaufschlagung durch die Spiralfeder 50 und der entfallenen Gegenkraft des Schädelknochens 88 durch den Stempel 60 in distaler Richtung kraftbeaufschlagt, weil jenseits des Schädelknochens 88 angeordnetes Weichgewebe 92 dem Stempel 60 keinen ausreichenden Widerstand entgegenzusetzen vermag (Figur 5).

Eine innenseitig am Schädelknochen 88 anliegende Knochenhaut 94 wird aufgrund des sich in distaler Richtung bewegenden Knochensegmentes 90 von den Zähnen 24 der Schneideinrichtung 26 wegbewegt. Darüber hinaus wird etwas Weichgewebe 92 in distaler Richtung verdrängt. Bei bestimmungsgemäßem Gebrauch der Aufsatzvorrichtung 10 ist dadurch die Möglichkeit gegeben, eine Verletzungsgefahr der Knochenhaut 94 und des Weichgewebes 92 durch die Zähne 24 der Schneideinrichtung 12 zu verringern. Ermöglicht wird dies dadurch, dass die Spiralfeder 50 den Stempel 60 zumindest dann, wenn der Abstand des Stempels 60 von der Schneidkante 26 einen Mindestabstand überschreitet, der unter anderem durch die Dicke des Schädelknochens 88 vorgegeben ist, mit einer in distaler Richtung wirkenden Kraft beaufschlagt und damit die Bewegung des Knochensegmentes 90 in distaler Richtung bewirkt.

Der durch den Ringflansch 56 definierte distale Anschlag 78 der Vorschubeinrichtung 34 sorgt dafür, dass die Bewegung des Stempels 60 und damit des Knochensegmentes 90 in distaler Richtung begrenzt ist (Figur 5). Infolgedessen ist auch die Eindringtiefe des Knochensegmentes 90 ins Innere des Schädels 84 begrenzt, so dass eine Gefahr der Verletzung des Weichgewebes 92 und der Knochenhaut 94 durch das Knochensegment 90 minimiert werden kann.

Weil sich der distale Anschlag 78 aufgrund der bestehenden Schraubverbindung zwischen der Schneideinrichtung 12 und der Vorschubeinrichtung 34 während des Schneidvorganges ebenfalls relativ zur Schneidkante 26 in proximaler Richtung bewegt, kann darüber hinaus der Vorschub des Stempels 60 nach dem Durchschneiden des Schädelknochens 88 minimiert werden.

Dies ist allerdings keine Voraussetzung für die Funktion der Aufsatzvorrichtung 10. Bei einer Variante der Aufsatzvorrichtung 10 könnte der Vorschubkörper 38 zum Beispiel außenseitig eine Rippe aufweisen, die in eine innenseitig am Schneidkörper 18 angeordnete Ringnut eingreift. In diesem Fall bestünde keine Schraubverbindung zwischen der Schneideinrichtung 12 und der Vorschubeinrichtung 34, und lineare Bewegung der Schneideinrichtung 12 relativ zur Vorschubeinrichtung 34 wäre nicht möglich. Dementsprechend fiele der Vorschubweg des Stempels 60 und damit des Knochensegmentes 90 nach dem Durchschneiden des Schädelknochens 88 in distaler Richtung größer aus als bei der Aufsatzvorrichtung 10. Gleichwohl kann damit die Knochenhaut 94 und das Weichgewebe 92 von den Zähnen 24 der Schneideinrichtung 12 wegbewegt werden.

Bei weiteren Modifikationen der Aufsatzvorrichtung 10 kann anstelle der Spiralfeder 50 ein andersartiges elastisches Element 52 zum Einsatz kommen, beispielsweise eine Blattfeder, eine Gasdruckfeder oder eine elastische Gummimanschette.

Bei einer Variante der Aufsatzvorrichtung 10 liegt der Stempel 60 nicht während des Schneidvorganges am Schädelknochen 88 an, sondern er wird erst nach dem Durchschneiden des Schädelknochens 88 durch die Spiralfeder 50 kraftbeaufschlagt.

Um festzustellen, ob der Schädelknochen 88 durchgeschnitten ist, kann die Aufsatzvorrichtung 10 eine separate Detektionseinrichtung umfassen, die beispielsweise mechanisch und/oder optisch funktioniert.

Anstelle der Kraftbeaufschlagungseinrichtung 48, die das elastische Element 52 umfasst, kann auch eine hydraulisch und/oder pneumatisch und/oder elastisch arbeitende Kraftbeaufschlagungseinrichtung zum Einsatz kommen.

Im Rahmen der beigefügten Patentansprüche ist ebenfalls möglich, dass eine Kraftbeaufschlagungseinrichtung zum Einsatz kommt, die nicht unmittelbar wie die Spiralfeder 50 auf den Stempel 60 einwirkt, sondern nur mittelbar. Eine derartige Kraftbeaufschlagungseinrichtung kann beispielsweise eine Düse umfassen, aus der zur Kraftbeaufschlagung des Stempels 60 dann ein Fluid wie zum Beispiel Pressluft ausströmt, wenn der Schädelknochen 88 mittels der Schneideinrichtung 12 durchgeschnitten ist.

Die chirurgische Vorrichtung zum Durchschneiden eines Knochens 11 kann über die Aufsatzvorrichtung 10 hinaus weitere Aufsatzvorrichtungen umfassen, die sich beispielsweise hinsichtlich des Durchmessers des Schneidkörpers 18 und damit des Durchmessers der am Schädel 84 erzeugbaren Schnittlinie unterscheiden. Derartige Aufsatzvorrichtungen kommen ebenso wie die Aufsatzvorrichtung 10 vormontierte und austauschbare Baueinheiten bilden, die sich auf einfache und bekannte Weise an die Antriebseinheit 80 anschließen oder von dieser abkoppeln lassen.

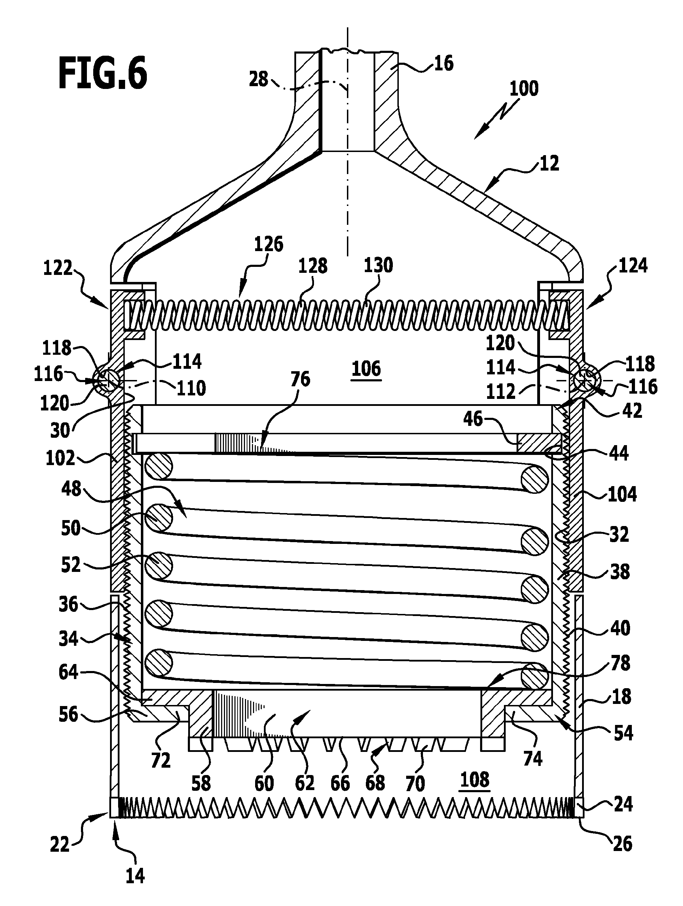

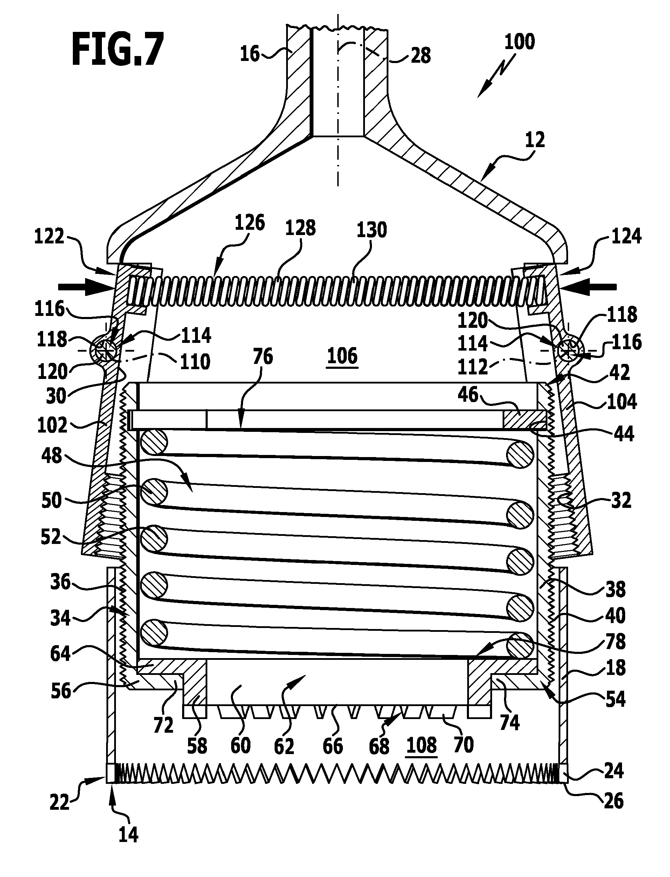

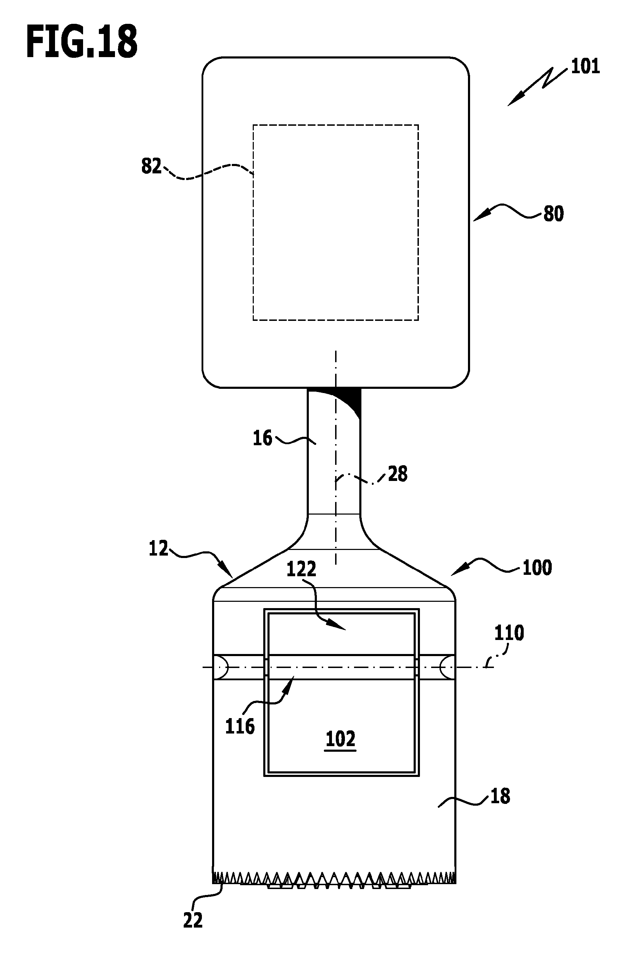

Eine weitere Ausführungsform einer Aufsatzvorrichtung wird nachfolgend beschrieben. Sie ist in den Figuren 6 und 7 dargestellt und dort mit dem Bezugszeichen 100 belegt. Die Aufsatzvorrichtung 100 kann mit der bereits erwähnten Antriebseinheit 80 zusammenwirken, um eine zweite Ausführungsform einer chirurgischen Vorrichtung zum Durchschneiden eines Knochens auszubilden, die in Figur 18 dargestellt und dort mit dem Bezugszeichen 101 belegt ist. Darüber hinaus kann die Aufsatzvorrichtung 100 ebenso wie die Aufsatzvorrichtung 10 als Zubehör oder Ergänzungsvorrichtung für weitere Antriebseinheiten verwendet werden und mit diesen weitere Ausführungsformen einer chirurgischen Vorrichtung zum Durchschneiden eines Knochens bilden.

Die Aufsatzvorrichtung 100 ist weitgehend bauartgleich mit der Aufsatzvorrichtung 10, weswegen für identische und/oder gleichwirkende Bauteile der Aufsatzvorrichtung 100 dieselben Bezugszeichen verwendet werden wie für die Aufsatzvorrichtung 10. Bezüglich dieser Bauteile wird zur Vermeidung von Wiederholungen auf die voranstehenden Erläuterungen Bezug genommen. Die vorstehend beschriebenen Vorteile der Aufsatzvorrichtung 10 können mit der Aufsatzvorrichtung 100 ebenfalls erzielt werden.

Bei der Aufsatzvorrichtung 100 weist der Schneidkörper 18 an seinem proximalen Endabschnitt vor der Verjüngung der Schneideinrichtung 12 zum Schaft 16 zwei Wandsegmente 102 und 104 auf. Diese liegen einander, bezogen auf die Achse 28, diametral gegenüber und erstrecken sich jeweils über ungefähr ein Achtel des Umfanges des Schneidkörpers 18. Dementsprechend umfasst der Schneidkörper 18 zwei weitere Wandsegmente, von denen in der Zeichnung lediglich ein Wandsegment 106 zu sehen ist, die sich jeweils über ungefähr drei Achtel des Schneidkörpers 18 erstrecken. Das Wandsegment 106 sowie das nicht gezeigte Wandsegment sind mit einem Wandsegment 108 des Schneidkörpers 18 einstückig verbunden, welches den distalen Endabschnitt des Schneidkörpers 18 bildet und an dem die Schneidelemente 22 angeordnet sind.

An dem Wandsegment 106 sowie dem nicht gezeigten Wandsegment sind die Wandsegmente 102 und 104 beweglich und insbesondere schwenkbar um zwei senkrecht zur Achse 28 ausgerichtete und senkrecht zur Zeichenebene dargestellte Achsen 110 beziehungsweise 112 gelagert. Zur schwenkbaren Lagerung der Wandsegmente 102 und 104 weist die Aufsatzvorrichtung 100 erste Lagerelemente 114 sowie zweite Lagerelemente 116 auf, die mit den ersten Lagerelementen 114 zusammenwirken. Die ersten Lagerelemente 114 sind als Durchbrechungen 118 ausgebildet und die zweiten Lagerelemente 116 als Lagerbolzen 130. Hiervon sind die Durchbrechungen 118 an den Wandsegmenten 102 und 104 und die Lagerbolzen 120 an dem Wandsegment 106 sowie an dem nicht gezeigten Wandsegment angeordnet.

Die Wandsegmente 102 und 104 weisen an ihrer jeweiligen Innenseite 30 das Innengewinde 32 der Schneideinrichtung 12 auf, so dass sich das Innengewinde 32 insgesamt über ungefähr ein Viertel des Umfanges des Schneidkörpers 18 erstreckt. Wie vorstehend im Fall der Aufsatzvorrichtung 10 erläutert, steht die Schneideinrichtung 12 über das Innengewinde 32 mit dem Außengewinde 36 der Vorschubeinrichtung 34 in Eingriff (Figur 6). Eine Stellung, in der das Innengewinde 32 mit dem Außengewinde 36 in Eingriff steht, wird als Betriebsstellung der Schneideinrichtung 12 bezeichnet, in welcher die Aufsatzvorrichtung 100 wie vorstehend im Fall der Aufsatzvorrichtung 10 beschrieben eingesetzt werden kann.

Durch Kraftbeaufschlagung ihrer proximalen Enden 122 und 124 senkrecht zur Achse 28 und vorliegend in der Zeichenebene, symbolisiert durch die Pfeile in Figur 7, können die Wandsegmente 102 und 104 relativ zum Rest des Schneidkörpers 18 verschwenkt werden. Die Richtung der Kraftbeaufschlagung ist vorliegend derart, dass die proximalen Enden 122 und 124 aufeinander zu bewegt werden, was es erlaubt, das Innengewinde 32 der Schneideinrichtung 12 außer Eingriff mit dem Außengewinde 36 der Vorschubeinrichtung 34 zu bringen. Dies wird als Lösestellung der Schneideinrichtung 12 bezeichnet. In der Lösestellung der Schneideinrichtung 12 kann die Vorschubeinrichtung 34 auf einfache Weise relativ zur Schneideinrichtung 12 versetzt werden. Dies ist beispielsweise vor oder nach Gebrauch der Schneideinrichtung 12 nützlich, um die Vorschubeinrichtung 34 für einen bevorstehenden oder erneuten Einsatz der Aufsatzvorrichtung 100 relativ zur Schneidkante 26 geeignet anzuordnen. Die Schneideinrichtung 12 der Aufsatzvorrichtung 100 ist insbesondere manuell von der Betriebsstellung in die Lösestellung überführbar, was der Aufsatzvorrichtung 100 eine einfache Handhabbarkeit verleiht. Außerdem weist die Aufsatzvorrichtung 100 eine Rückstelleinrichtung 126 auf, welche ein elastisches Element 128 in Form einer Spiralfeder 130 umfasst. Die Spiralfeder 130 ist zwischen den proximalen Enden 122 und 124 der Wandsegmente 102 beziehungsweise 104 senkrecht zur Achse 28 angeordnet und insbesondere in der Betriebsstellung der Schneideinrichtung 12 zwischen diesen vorgespannt. Das Überführen der Schneideinrichtung 12 von der Betriebsstellung in die Lösestellung erfolgt daher entgegen der Wirkung der Spiralfeder 130, mittels derer die Schneideinrichtung 12 selbsttätig wieder von der Lösestellung in die Betriebsstellung überführbar ist. Darüber hinaus ist es selbstverständlich möglich, die Schneideinrichtung 12 auch manuell von der Lösestellung in die Betriebsstellung zu überführen.

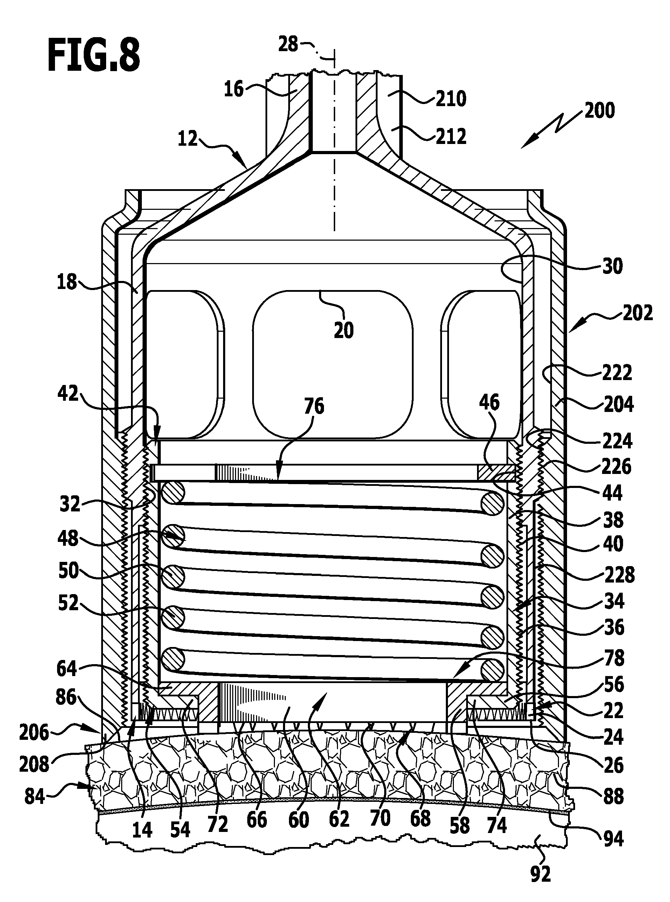

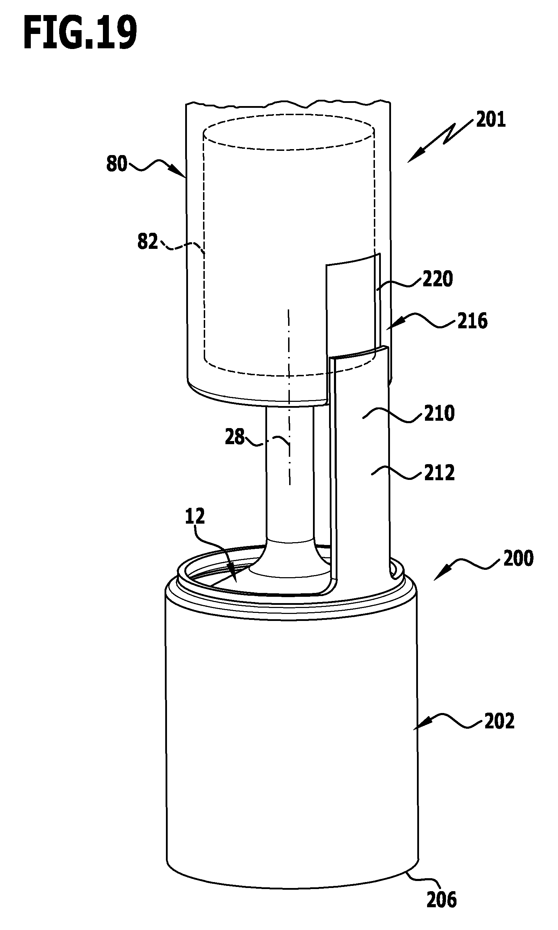

Eine dritte und bevorzugte Ausführungsform einer Aufsatzvorrichtung wird nachfolgend beschrieben und stellt eine erfindungsgemäße Aufsatzvorrichtung dar. Diese ist in den Figuren 8 bis 10 schematisch dargestellt und dort insgesamt mit dem Bezugszeichen 200 belegt. Die Aufsatzvorrichtung 200 kann mit der bereits beschriebenen Antriebseinheit 80 zur Ausbildung einer dritten, bevorzugten Ausführungsform der chirurgischen Vorrichtung zum Durchschneiden eines Knochens zusammenwirken, die in Figur 19 dargestellt und dort mit dem Bezugszeichen 201 belegt ist. Die Aufsatzvorrichtung 200 kann darüber hinaus als Zubehör oder Ergänzungsvorrichtung für weitere Antriebseinheiten verwendet werden und mit diesen weitere bevorzugte Ausführungsformen einer erfindungsgemäßen Vorrichtung zum Durchschneiden eines Knochens bilden.

Die Aufsatzvorrichtung 200 ist zu großen Teilen bauartgleich mit der Aufsatzvorrichtung 10, weswegen für identische und/oder gleichwirkende Bauteile der Aufsatzvorrichtung 200 dieselben Bezugszeichen wie für diejenigen der Aufsatzvorrichtung 10 verwendet werden. Bezüglich dieser Bauteile wird zur Vermeidung von Wiederholungen auf die voranstehenden Erläuterungen Bezug genommen. Die voranstehenden Vorteile der Aufsatzvorrichtung 10 können mit der Aufsatzvorrichtung 200 ebenfalls erzielt werden.

Die Aufsatzvorrichtung 200 weist eine Abstützeinrichtung 202 auf, die koaxial zur Schneideinrichtung 12 und zur Vorschubeinrichtung 34 ausgerichtet ist und mittels eines hülsenartigen und insbesondere ungefähr hohlzylindrischen Abstützkörpers 204 sowohl die Schneideinrichtung 12 als auch die Vorschubeinrichtung 34 umgibt. Das distale Ende des Abstützkörpers 204 bildet ein ringförmiges und insbesondere kreisförmiges Abstützglied 206 in Form einer in distaler Richtung etwas spitz zulaufenden Stützkante 208.

Der Abstützkörper 204 ist insgesamt weitgehend rotationssymmetrisch bezüglich der Achse 28 ausgebildet, wobei die Rotationssymmetrie durch ein vom proximalen Ende des Abstützkörpers 204 in proximaler Richtung abstehendes Fixierglied 210 aufgehoben wird. Das Fixierglied 210 verläuft parallel und in Abstand zum Schaft 16 der Schneideinrichtung 12 und ist als flacher Materialstreifen 212 ausgebildet.

Die Antriebseinheit 80 umfasst eine mit dem Fixierglied 210 zusammenwirkende Fixiereinrichtung 216 (Figur 19), die außenseitig angeordnet ist und eine parallel zur Achse 28 verlaufende Ausnehmung 220 aufweist, in welcher das proximale Ende des Fixiergliedes 210 parallel zur Achse 28 geführt wird, wenn die Aufsatzvorrichtung 200 an die Antriebseinheit 80 angeschlossen ist.

An seiner Innenseite 222, die dem Schneidkörper 18 zugewandt ist, weist der Abstützkörper 204 ein Innengewinde 224 auf, das mit einem Außengewinde 226 in Eingriff steht, welches an einer Außenseite 228 des Schneidkörpers 18 angeordnet ist. Die Schneideinrichtung 12 und die Abstützeinrichtung 202 bilden auf diese Weise eine Schraubverbindung aus, die eine Schraubführung definiert, so dass die Abstützeinrichtung 202 relativ zur Schneideinrichtung 12 sowohl drehbeweglich als auch linear beweglich ausgebildet ist, wobei die lineare Bewegung parallel zur Achse 28 sowohl in proximaler als auch in distaler Richtung erfolgen kann.

Zur Anwendung der Aufsatzvorrichtung 200 ist diese derart auf der Außenseite 86 des Schädelknochens 88 aufzusetzen, dass zusätzlich zum Stempel 60 der Vorschubeinrichtung 34 auch die Stützkante 208 der Abstützeinrichtung 202 am Schädelknochen 88 anliegt (Figur 4). Bei Kraftbeaufschlagung der Aufsatzvorrichtung 200 in distaler Richtung gerät dadurch der Abstützkörper 204 in Eingriff mit dem Schädelknochen 88.

Wird die Schneideinrichtung 12 wie vorstehend beschrieben in Drehung versetzt, bleibt die mit dem Schädelknochen 88 in Eingriff stehende Abstützeinrichtung 202 drehbeweglich zur Schneideinrichtung 12, denn sie wird mittels der Fixiereinrichtung 216 daran gehindert, zusammen mit der Schneideinrichtung 12 zu rotieren. Aufgrund der bestehenden Schraubverbindung zwischen der Schneideinrichtung 12 und der Abstützeinrichtung 202 bewegt sich letztere relativ zur Schneidkante 26 in proximaler Richtung, wobei die Bewegung der Abstützeinrichtung 202 durch das in der Ausnehmung 220 geführte Fixierglied 210 geführt wird.

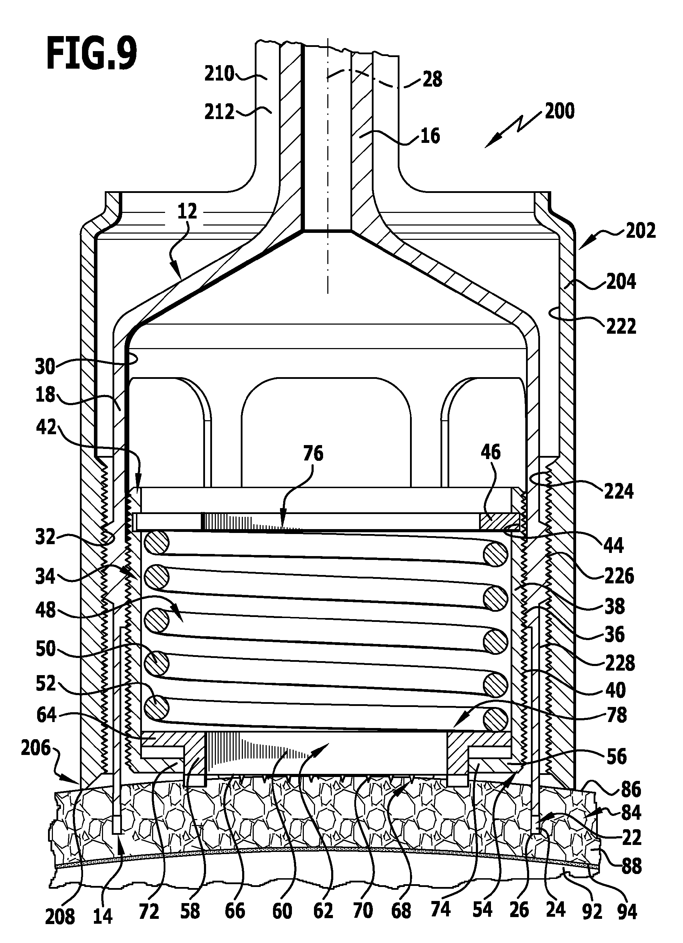

Das Außengewinde 36 der Vorschubeinrichtung 34, das Innengewinde 32 und das Außengewinde 226 der Schneideinrichtung 12 sowie das Innengewinde 224 der Abstützeinrichtung 202 weisen jeweils denselben Gewindegang auf, so dass sich die Vorschubeinrichtung 34 und die Abstützeinrichtung 202 relativ zur Schneidkante 26 um dieselbe Strecke in proximaler Richtung bewegen. Infolgedessen dreht sich die Schneideinrichtung 12 zwischen der Vorschubeinrichtung 34 und der Abstützeinrichtung 202, und die Vorschubeinrichtung 34 und die Abstützeinrichtung 202 sind verdrehgesichert zueinander ausgebildet (Figur 9).

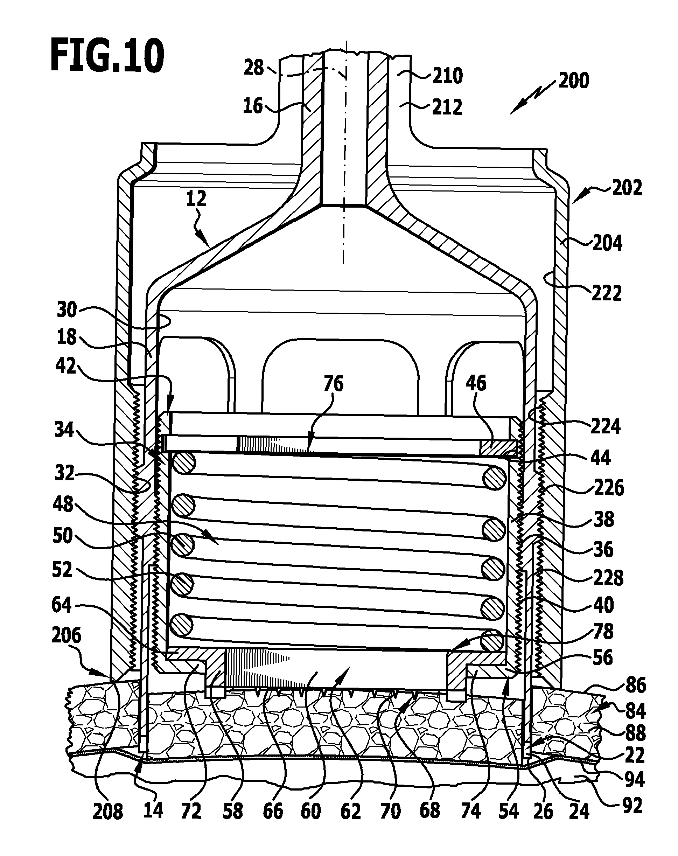

Während des Schneidvorganges dient die Abstützeinrichtung 202 zur Abstützung der gesamten Aufsatzvorrichtung 200 und damit auch der chirurgischen Vorrichtung 201 am Schädelknochen 88. Ist der Schädelknochen 88 mittels der Schneideinrichtung 12 durchgeschnitten (Figur 10), kann auf diese Weise die Gefahr, dass die Aufsatzvorrichtung 200 als Ganzes in den Schädel 84 eindringt und infolgedessen Verletzungen des Weichgewebes 92 und der Knochenhaut 94 hervorruft, verringert werden.

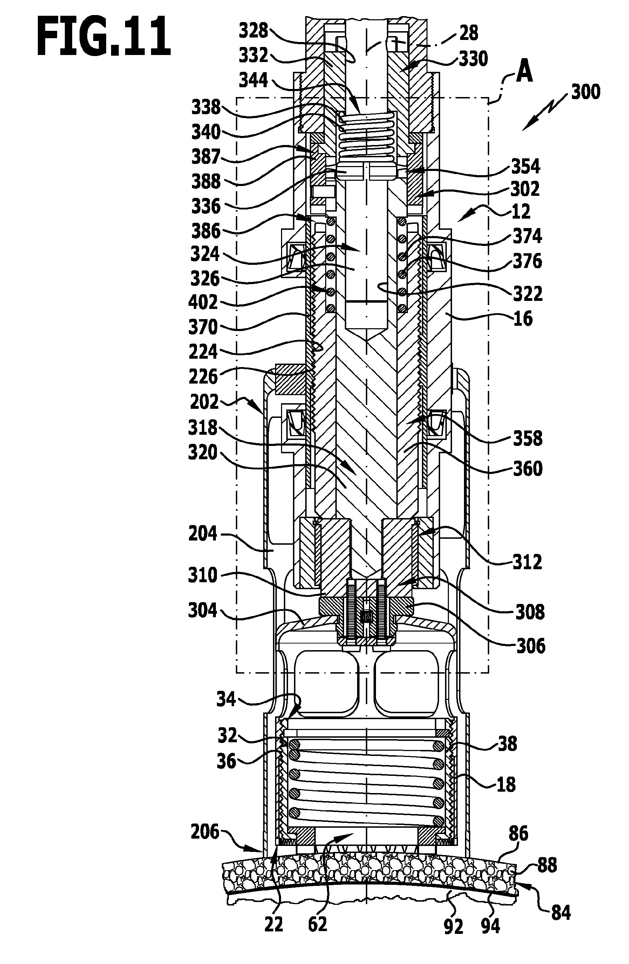

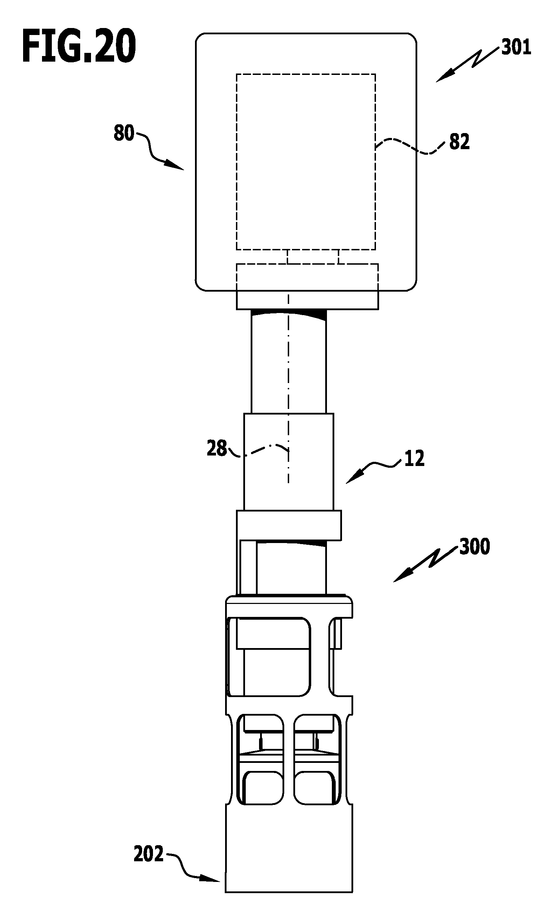

Eine nachfolgend beschriebene vierte, bevorzugte Ausführungsform einer Aufsatzvorrichtung stellt eine weitere erfindungsgemäße Aufsatzvorrichtung dar. Diese ist in den Figuren 11 bis 16 dargestellt und dort insgesamt mit dem Bezugszeichen 300 belegt. Die Aufsatzvorrichtung 300 kann zusammen mit der bereits erwähnten Antriebseinheit 80 eine vierte, bevorzugte Ausführungsform einer chirurgischen Vorrichtung zum Durchschneiden eines Knochens ausbilden, die eine erfindungsgemäße chirurgische Vorrichtung darstellt. Diese ist in Figur 20 schematisch dargestellt und dort mit dem Bezugszeichen 301 belegt ist. Darüber hinaus kann die Aufsatzvorrichtung 300 als Zubehör oder Ergänzungsvorrichtung für weitere Antriebseinheiten verwandt werden und mit diesen weitere bevorzugte Ausbildungsformen einer erfindungsgemäßen chirurgischen Vorrichtung zum Durchschneiden eines Knochens bilden.

Die Aufsatzvorrichtung 300 weist eine Vielzahl von Bauteilen auf, die identisch und/oder gleichwirkend zu den Bauteilen der Aufsatzvorrichtung 10 ausgebildet sind und darüber hinaus eine Mehrzahl von Bauteilen, die identisch und/oder gleichwirkend zu Bauteilen der Aufsatzvorrichtung 200 ausgebildet sind. Deswegen werden die Bauteile, die identisch oder gleichwirkend zu Merkmalen der Aufsatzvorrichtungen 10 oder 200 ausgebildet sind, mit denselben Bezugszeichen wie die entsprechenden Bauteile der Aufsatzvorrichtungen 10 und 200 bezeichnet. Bezüglich dieser Bauteile wird zur Vermeidung von Wiederholungen auf die voranstehenden Erläuterungen Bezug genommen.

Die voranstehend beschriebenen Vorteile der Aufsatzvorrichtungen 10 und 200 können mit der Aufsatzvorrichtung 300 ebenfalls erzielt werden.

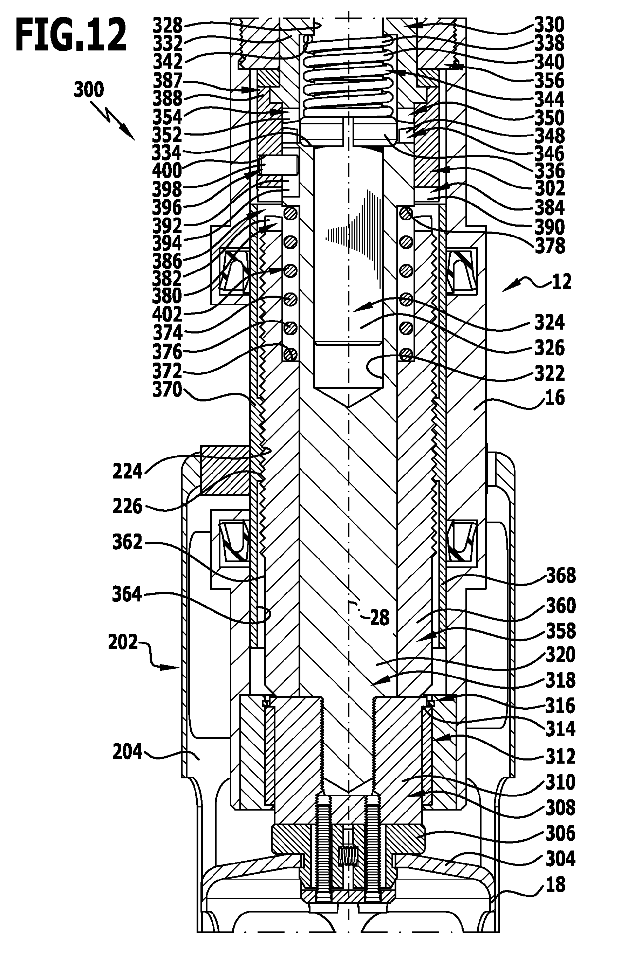

Nachfolgend wird unter Verweis insbesondere zunächst auf die Figuren 11 und 12 der Aufbau der Schneideinrichtung 12 der Aufsatzvorrichtung 300 näher erläutert:

Der Schaft 16 der Schneideinrichtung 12 ist bei der Aufsatzvorrichtung 300 nicht mit dem Schneidkörper 18 verbunden, sondern er bildet zumindest teilweise ein Gehäuse für eine Getriebeeinrichtung 302, welche von der Schneideinrichtung 12 umfasst wird. Antriebskräfte auf die Schneideinrichtung 12 werden dadurch bei der Aufsatzvorrichtung 300 im Gegensatz zu den Aufsatzvorrichtungen 10, 100 und 200 nicht über den Schaft 16 übertragen, sondern durch innerhalb der Schaftes 16 angeordnete Bauteile der Getriebeeinrichtung 302, die nachfolgend beschrieben werden.

Der Schneidkörper 18 der Aufsatzvorrichtung 312 weist an seinem proximalen Ende eine Deckenwand 304 auf, in die ein Verbindungsteil 306 eingesetzt ist, das mit einem ersten Getriebeteil 308 in Form einer Buchse 310 drehfest verbunden ist, vorliegend durch Verschraubung, wobei die Verbindung auch auf andere Weise erfolgen kann. Die Buchse 310 ist ringförmig von einem zweiten Getriebeteil 312 umgeben, welches seinerseits vom Schaft 16 umgeben ist und mit diesem fest verbunden ist. Das zweite Getriebeteil 312 weist an seinem proximalen Ende eine ringförmige Schulter 314 auf, die einen Anschlag 316 für die Buchse 310 bildet, welche längs der Achse 28 relativ zum zweiten Getriebeteil 312 und zum Schaft 16 verschiebbar ist.

Die Buchse 310 ist mit einem dritten Getriebeteil 318, das als Abtriebswelle 320 ausgebildet ist, drehfest verbunden, vorliegend durch Verschraubung, wobei die Verbindung auch auf andere Weise erfolgen kann. An ihrem proximalen Ende ist die Abtriebswelle 320 näherungsweise hohlzylindrisch ausgebildet und umfasst eine zentrale Aufnahme 322, in welcher ein viertes Getriebeteil 324 in Form eines Führungsbolzens 326 mit seinem distalen Ende eingreift.

Mit seinem proximalen Ende greift der Führungsbolzen 326 in eine zentrale Aufnahme 328 ein, die an einem fünften Getriebeteil 330, welches als näherungsweise hohlzylindrische Antriebswelle 332 ausgestaltet ist, gebildet/ist. Ein sechstes Getriebeteil 356 umgibt die Antriebswelle 332, ist seinerseits vom Schaft 16 umgeben und mit diesem verbunden.

Die Antriebswelle 332 ist auf an sich bekannte und deswegen in der Zeichnung nicht dargestellte Weise mit einer an sich bekannten und deswegen in der Zeichnung ebenfalls nicht dargestellten Anschlusseinrichtung der Aufsatzvorrichtung 300 verbunden, zu deren Anschließen beispielsweise an die Antriebseinheit 80.

Die Abtriebswelle 320 weist an ihrem proximalen Ende eine Schulter 334 auf, an der der Führungsbolzen 326 mittels eines ihn umgebenden Ringkragens 336 anliegt. In proximaler Richtung stützt sich auf dem Ringkragen ein elastisches Element 338 in Form einer Spiralfeder 340 ab, deren proximales Ende sich an einer Schulter 342 in der Aufnahme 328 der Antriebswelle 332 abstützt und eine Rückstelleinrichtung 344 der Aufsatzvorrichtung 300 bildet, was unten noch näher erläutert wird.

Darüber hinaus umfasst die Abtriebswelle 320 an ihrem proximalen Ende eine Mehrzahl von ersten Kupplungsgliedern 346 in Form von hervorspringenden Zähnen 348, die mit einer Mehrzahl von zweiten Kupplungsgliedern 350 zusammenwirken können, die ebenfalls als hervorspringende Zähne 352 ausgebildet sind und am distalen Ende der Antriebswelle 332 angeordnet sind. Die Zähne 348 und 352 bilden gemeinsam eine erste Kupplungseinrichtung 354 der Aufsatzvorrichtung.

In ihrem distalen, das heißt der Buchse 310 zugewandeten Bereich ist die Abtriebswelle 320 von einem siebten Getriebeteil 358 in Form einer Abtriebshülse 360 umgeben.

Die Abtriebshülse 360 weist an ihrer Außenseite 362 das Außengewinde 226 der Schneideinrichtung 12 auf. Das Außengewinde 226 steht mit dem Innengewinde 224 der Abstützeinrichtung 202 in Eingriff, das an einer Innenseite 364 eines hülsenförmigen und insbesondere hohlzylindrischen Gewindekörpers 370 angeordnet ist. Der Gewindekörper 370 wird von der Abstützeinrichtung 202 der Aufsatzvorrichtung 300 umfasst und ist mit dem proximalen Ende des Abstützkörpers 204 drehfest verbunden. Darüber hinaus liegt der Gewindekörper 370 innenseitig am Schaft 16 an, relativ zu dem er drehbeweglich und verschiebbar ausgebildet ist.

Die Schneideinrichtung 12 und die Abstützeinrichtung 202 der Aufsatzvorrichtung 300 bilden wie im Fall der vorstehend beschriebenen Aufsatzvorrichtung 200 eine Schraubverbindung aus, so dass die Abstützeinrichtung 202 relativ zur Schneideinrichtung 12 sowohl drehbeweglich ausgebildet als auch linear beweglich parallel zur Achse 28, und zwar sowohl in proximaler als auch in distaler Richtung.

Ungefähr auf Höhe des distalen Endes der Aufnahme 322 der Antriebswelle 320 weist die Abtriebshülse 360 eine Schulter 372 auf, an der sich ein elastisches Element 374 in Form einer Spiralfeder 376 mit ihrem distalen Ende abstützt. Das proximale Ende der Spiralfeder 376 stützt sich an einer Schulter 378 der Abtriebswelle 320 ab, welche in etwas Abstand zum proximalen Ende der Abtriebswelle 320 angeordnet ist.

An ihrem proximalen Ende weist die Abtriebshülse 360 eine Mehrzahl von dritten Kupplungsgliedern 380 auf, die als hervorspringende Zähne 382 ausgebildet sind und mit einer Mehrzahl von vierten Kupplungsgliedern 384 zusammenwirken können zur Ausbildung einer zweiten Kupplungseinrichtung 386 der Aufsatzvorrichtung 300. Die vierten Kupplungsglieder 384 werden von einem achten Getriebeteil 387 in Form einer Antriebshülse 388 umfasst und sind ebenfalls als hervorspringende Zähne 390 ausgebildet. Die Antriebshülse 388 umgibt das distale Ende der Antriebswelle 332 und das proximale Ende der Abtriebswelle 320 und ist seinerseits vom Schaft 16 umgeben.

Auf einer Außenseite weist die Abtriebswelle 320 zwischen den Zähnen 348 und der Schulter 378 eine Aufnahme 392 in Form eines Langloches 394 auf, in welches ein Kuppelglied 396 in Form eines Mitnehmerstiftes 398 eingreift, dessen anderes Ende in eine Aufnahme 400 eingreift, die nahe dem distalen Ende der Antriebshülse 388 gebildet ist. Im Langloch 394 ist der Mitnehmerstift 398 parallel zur Achse 28 verschieblich, und über den Mitnehmerstift 398 besteht zwischen der Abtriebswelle 320 und der Antriebshülse 388 eine drehfeste Verbindung.

Zum Durchschneiden des Schädelknochens 88 mit der Aufsatzvorrichtung 300 ist folgendermaßen vorzugehen:

Zunächst ist die Aufsatzvorrichtung 300, wie bereits erwähnt, über ihre am proximalen Ende angeordnete und in der Zeichnung nicht dargestellte Anschlusseinrichtung mit einer Antriebseinheit, beispielsweise der Antriebseinheit 80, zu verbinden, um die Antriebswelle 332 anzutreiben. Um den Schneidkörper 18 in eine Drehbewegung zu versetzen, ist die Antriebswelle 332 ebenfalls in eine Drehbewegung zu versetzen. Alternativ ist es selbstverständlich auch möglich, die Antriebswelle 332 oszillierend und/oder gemäß einem Pilgerschrittverfahren anzutreiben, wie es oben beschrieben wurde.

Zum Durchschneiden des Schädelknochens 88 ist die Aufsatzvorrichtung 300 auf die Außenseite 86 des Schädelknochens 88 aufzusetzen, so dass die Anlagefläche 66 des Stempels 60 und die Stützkante 208 der Abstützeinrichtung 202 am Schädelknochen 88 anliegen. Dies ist in der Figur 11 dargestellt, wobei die Aufsatzvorrichtung 300 lediglich lose am Schädelknochen 88 anliegend gezeigt ist, ohne dass sie vom Operateur mit einer in distaler Richtung wirkenden Kraft beaufschlagt wird.

Wie in Figur 11 und insbesondere in Figur 12 zu sehen ist, sind in diesem Fall die Zähne 348 und 352 sowie die Zähne 382 und 390 außer Eingriff, das heißt die erste Kupplungseinrichtung 354 und die zweite Kupplungseinrichtung 386 sind entkoppelt. Dies ist dadurch bedingt, dass im Falle der ersten Kupplungseinrichtung 354 die Spiralfeder 340 zwischen der Antriebswelle 332 und der Abtriebswelle 320 vorgespannt ist; über den Ringkragen 336 wird die Abtriebswelle 320 mit einer von der Antriebswelle 332 wegweisenden Kraft beaufschlagt. Im Falle der zweiten Kupplungseinrichtung 386 kraftbeaufschlagt die Spiralfeder 376 die Abtriebshülse 360 mit einer von der Antriebshülse 388 wegweisenden Kraft.

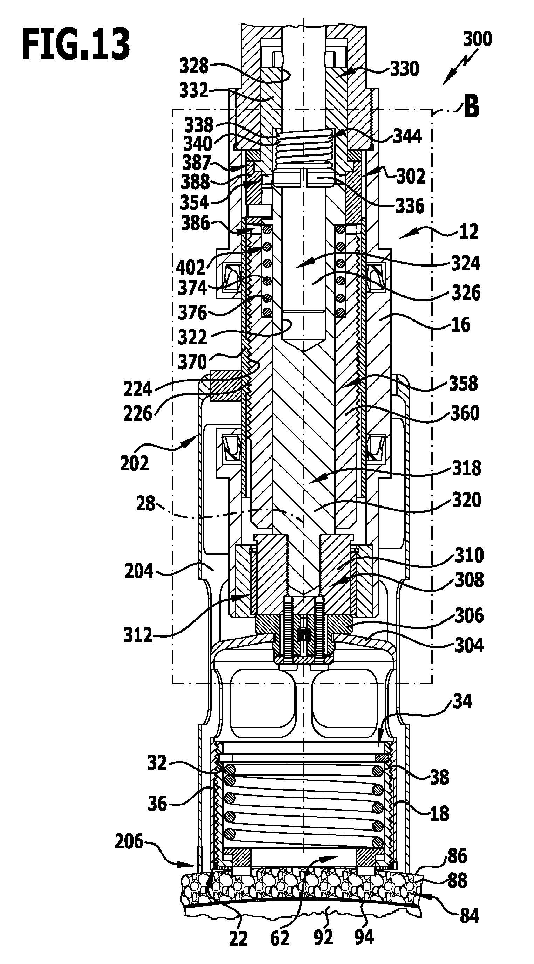

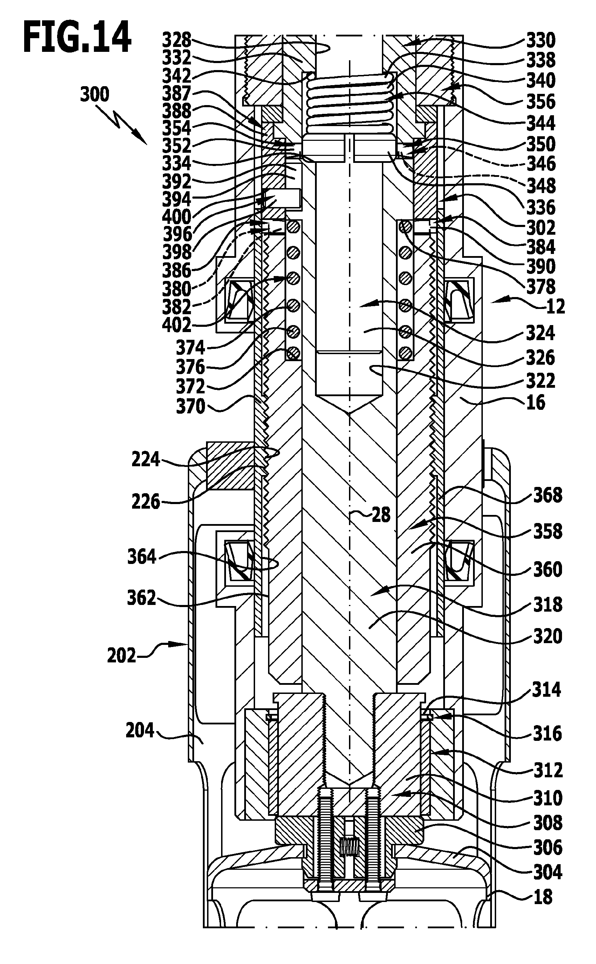

Durch Kraftbeaufschlagung der Aufsatzvorrichtung 300 in distaler Richtung entgegen der Gegenkraft des Schädelknochens 88 (Figuren 13 und 14) können sowohl die erste Kupplungseinrichtung 354 als auch die zweite Kupplungseinrichtung 386 miteinander in Eingriff gebracht werden. Im Falle der ersten Kupplungseinrichtung 354 erfolgt dies dadurch, dass der Schneidkörper 18 die Gegenkraft des Schädelknochens 88 in proximaler Richtung aufnimmt, vermittelt entweder durch die Vorschubeinrichtung 34 oder die Schneidelemente 22. Der Schneidkörper 18 überträgt die Gegenkraft des Schädelknochens 88 über das Verbindungsteil 306 und die Buchse 310 auf die Abtriebswelle 320, um die Zähne 348 mit den Zähnen 352 in Eingriff zu bringen. Dies erfolgt entgegen der Vorspannung der Spiralfeder 340, welche von der Abtriebswelle 320 vermittelt durch den Ringkragen 336 zusammengedrückt werden kann. Der Mitnehmerstift 398 wird dabei relativ zur Abtriebswelle 320 in distaler Richtung im Langloch 394 verschoben.

Auf die vorstehend beschriebene Weise ist die erste Kupplungseinrichtung 354 eingekoppelt, und eine Drehbewegung kann von der Antriebswelle 332 auf den Schneidkörper 18 zum Durchschneiden des Schädelknochens 88 mittels der Schneidelemente 22 übertragen werden.

Das Einkoppeln der zweiten Kupplungseinrichtung 386 erfolgt dadurch, dass der Abstützkörper 204 mit der Stützkante 208 die Gegenkraft des Schädelknochens 88 aufnimmt. Aufgrund der festen Verbindung des Abstützkörpers 204 mit dem Gewindekörper 370 wird die Abtriebshülse 360, die mit dem Gewindekörper 370 in Eingriff steht, in Richtung der Antriebshülse 388 verschoben.

Dies erfolgt solange, bis die Zähne 382 mit den Zähnen 390 in Eingriff stehen, entgegen der Wirkung der Spiralfeder 376.

Über den Mitnehmerstift 398 sind die Zähne 390 der zweiten Kupplungseinrichtung 386 mechanisch mit der ersten Kupplungseinrichtung 354 gekoppelt. Auf diese Weise ist möglich, eine Drehbewegung der Antriebswelle 332 über die erste Kupplungseinrichtung 354 auf die Abtriebswelle 320 und anschließend über den Mitnehmerstift 398 auf die Antriebshülse 388 zu übertragen, woraufhin deren Drehbewegung über die zweite Kupplungseinrichtung 386 auf die Abtriebshülse 360 übertragen werden kann.

Aufgrund der bestehenden Schraubverbindung zwischen der Abtriebshülse 360 und dem Gewindekörper 370 kann sich die Abstützeinrichtung 202 parallel zur Achse 28 relativ zur Schneideinrichtung 12 in proximaler Richtung bewegen, wie dies vorstehend bereits im Falle der Aufsatzvorrichtung 200 beschrieben wurde. Die Gewindegänge des Außengewindes 36 der Vorschubeinrichtung 34, des Innengewindes 32 der Schneideinrichtung 12, des Außengewindes 226 der Schneideinrichtung 12 sowie des Innengewindes 224 der Abstützeinrichtung 202 sind identisch ausgebildet, so dass beim Durchschneiden des Schädelknochens 88 die Vorschubeinrichtung 34 und die Abstützeinrichtung 202, die jeweils mit dem Schädelknochen 88 in Eingriff stehen, drehfest zueinander ausgebildet sind. Zwischen der Vorschubeinrichtung 34 und der Abstützeinrichtung 202 wird der Schädelknochen vom Schneidkörper 18 der Schneideinrichtung 12 durchschnitten.

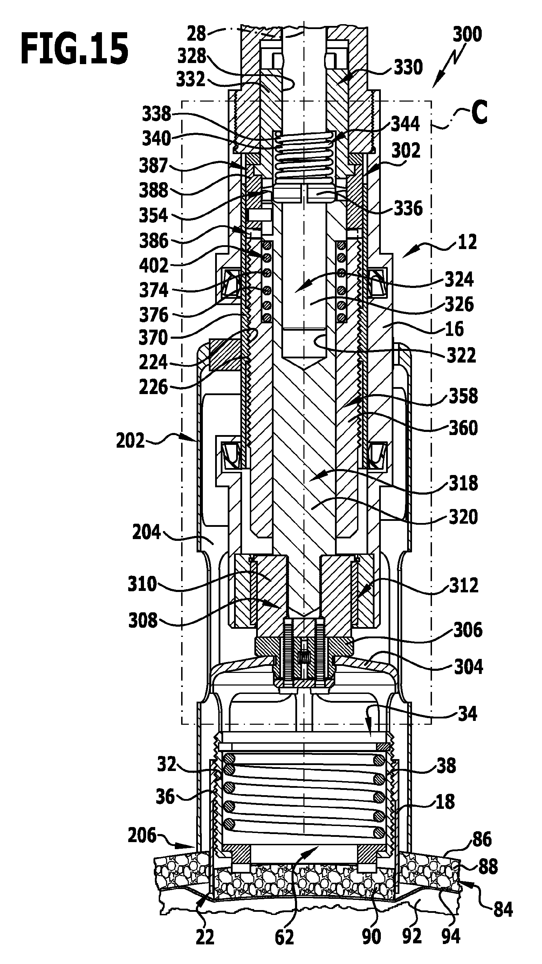

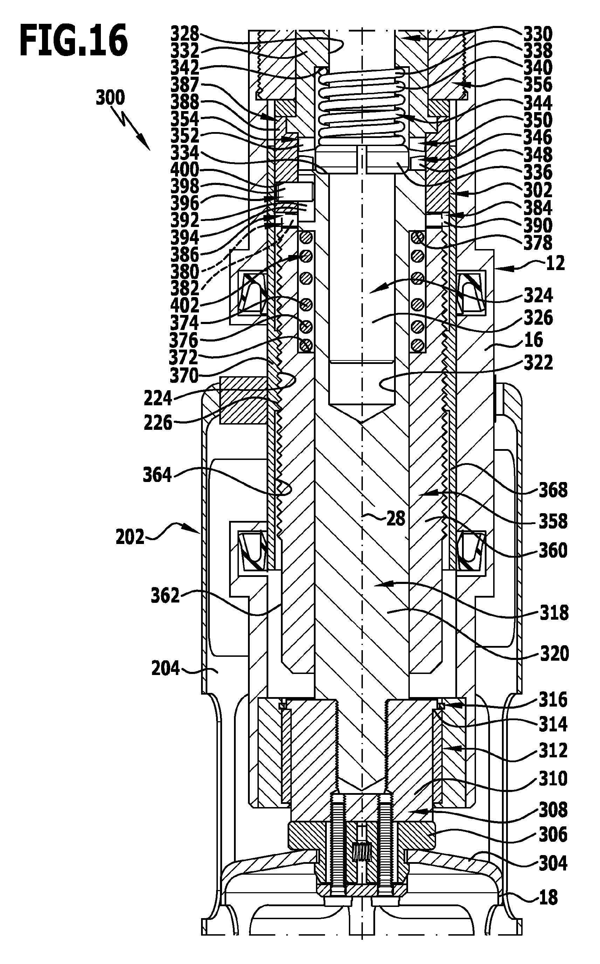

Wie bereits erwähnt, bildet die Spiralfeder 340 eine Rückstelleinrichtung 344 der Aufsatzvorrichtung 300. Nach dem Durchschneiden des Schädelknochens 88 (Figuren 15 und 16) entfällt die bis dahin vom Schädelknochen 88 ausgeübte Gegenkraft, so dass die Spiralfeder 340 aufgrund ihrer Vorspannung zwischen der Antriebswelle 332 und der Abtriebswelle 320 letztere so von der Antriebswelle 332 wegbewegen kann, dass die Zähne 348 und 352 außer Eingriff geraten. Dies bedeutet, dass nach dem Durchschneiden des Schädelknochens 88 die erste Kupplungseinrichtung 354 automatisch entkoppelt. Selbst wenn die Antriebswelle 332 nach dem Durchschneiden des Schädelknochens 88 noch eine Drehbewegung ausführt, wird diese nicht mehr auf den Schneidkörper 18 übertragen. Die Gefahr einer Verletzung der Knochenhaut 94 und des Weichgewebes 92 kann dadurch weitgehend verhindert werden. Der Anschlag 316 an dem zweiten Getriebeteil 312 sorgt dafür, dass der Schneidkörper 18 beim Entkoppeln der ersten Kupplungseinrichtung 354 nur so weit in den Schädel 84 eindringt, wie unbedingt erforderlich ist.

Auch nach dem Entkoppeln der ersten Kupplungseinrichtung 354 bleibt die zweite Kupplungseinrichtung 386 eingekoppelt (Figuren 15 und 16), und zwar solange, wie die Aufsatzvorrichtung 300 mit einer in distaler Richtung wirkenden Kraft beaufschlagt wird, die vom Schädelknochen 88 kompensiert werden kann. Nach dem Entfernen der Aufsatzvorrichtung 300 vom Schädelknochen 88 geraten auch die Zähne 382 und 390 aufgrund der Rückstellkraft der Spiralfeder 376, die damit ebenfalls eine Rückstelleinrichtung 402 der Aufsatzvorrichtung 300 bildet, außer Eingriff.

## Patentansprüche

1. Aufsatzvorrichtung (10; 100; 200; 300) für eine chirurgische Vorrichtung, wobei die Aufsatzvorrichtung (10; 100; 200; 300) eine Schneideinrichtung (12) mit mindestens einem mindestens eine Schneidkante (26) bildenden Schneidelement (22) zur Erzeugung mindestens einer Schnittlinie an einem Knochen (88) umfasst, wobei die Aufsatzvorrichtung (10; 100; 200; 300) eine Vorschubeinrichtung (34) aufweist, die mindestens ein Vorschubglied (62) umfasst, welches relativ zu dieser mindestens einen Schneidkante (26) beweglich ausgebildet ist und dadurch relativ zu dieser einen variablen Abstand einnehmen kann, sowie eine Kraftbeaufschlagungseinrichtung (48), welche so ausgebildet ist, dass das mindestens eine Vorschubglied (62) mit einer in distaler Richtung wirkenden Kraft beaufschlagbar ist, zumindest sofern der Abstand einen Mindestabstand überschreitet, und wobei die Aufsatzvorrichtung (200; 300) eine Abstützeinrichtung (202) aufweist mit mindestens einem an den Knochen (88) anlegbaren Abstützglied (206), **dadurch gekennzeichnet, dass** die Abstützeinrichtung (202) verdrehgesichert zur Vorschubeinrichtung (34) ausgebildet ist.

2. Aufsatzvorrichtung nach Anspruch 1, **dadurch gekennzeichnet, dass** das mindestens eine Vorschubglied (62) mindestens ein sich in distaler Richtung erstreckendes Eingriffselement (68) zum Eingreifen in den Knochen (88) umfasst.

3. Aufsatzvorrichtung nach Anspruch 1 oder 2, **dadurch gekennzeichnet, dass** die Vorschubeinrichtung (34) drehbeweglich zur Schneideinrichtung (12) ausgebildet ist.

4. Aufsatzvorrichtung nach einem der voranstehenden Ansprüche, **dadurch gekennzeichnet, dass** das mindestens eine Vorschubglied (62) verdrehgesichert zur Vorschubeinrichtung (34) ausgebildet ist.

5. Aufsatzvorrichtung nach einem der voranstehenden Ansprüche, **dadurch gekennzeichnet, dass** die Schneideinrichtung (12) mit der Vorschubeinrichtung (34) eine Schraubverbindung ausbildet.

6. Aufsatzvorrichtung nach einem der voranstehenden Ansprüche, **dadurch gekennzeichnet, dass** die Abstützeinrichtung (202) drehbeweglich zur Schneideinrichtung (12) ausgebildet ist.

7. Aufsatzvorrichtung nach einem der voranstehenden Ansprüche , **dadurch gekennzeichnet, dass** die Abstützeinrichtung (202) mit der Schneideinrichtung (12) eine Schraubverbindung ausbildet.

8. Aufsatzvorrichtung nach einem der voranstehenden Ansprüche, **dadurch gekennzeichnet, dass** die Aufsatzvorrichtung (300) eine der Schneideinrichtung (12) zugeordnete erste Kupplungseinrichtung (354) aufweist mit einem mit dem mindestens einen Schneidelement (22) in Wirkverbindung stehenden ersten Kupplungsglied (346) und einem antreibbaren zweiten Kupplungsglied (350), das mit dem ersten Kupplungsglied (346) zusammenwirkt.

9. Aufsatzvorrichtung nach Anspruch 8, **dadurch gekennzeichnet, dass** das erste Kupplungsglied (346) mit dem zweiten Kupplungsglied (350) durch Kraftbeaufschlagung des mindestens einen Schneidelementes (22) und/oder des mindestens einen Vorschubgliedes (34) in proximaler Richtung in Wirkverbindung bringbar ist.

10. Aufsatzvorrichtung nach Anspruch 8 oder 9, **dadurch gekennzeichnet, dass** die Aufsatzvorrichtung (300) eine Rückstelleinrichtung (344) umfasst, entgegen deren Wirkung das erste Kupplungsglied (346) mit dem zweiten Kupplungsglied (350) in Wirkverbindung bringbar ist.

11. Aufsatzvorrichtung nach einem der voranstehenden Ansprüche , **dadurch gekennzeichnet, dass** die Aufsatzvorrichtung (300) eine der Abstützeinrichtung (202) zugeordnete zweite Kupplungseinrichtung (386) aufweist mit einem mit dem mindestens einen Abstützglied (206) in Wirkverbindung stehenden dritten Kupplungsglied (380) und einem antreibbaren vierten Kupplungsglied (384), das mit dem dritten Kupplungsglied (380) zusammenwirkt.

12. Aufsatzvorrichtung nach Anspruch 11, **dadurch gekennzeichnet, dass** das dritte Kupplungsglied (380) mit dem vierten Kupplungsglied (384) durch Kraftbeaufschlagung des mindestens einen Abstützgliedes (206) in proximaler Richtung in Wirkverbindung bringbar ist.

13. Aufsatzvorrichtung nach Anspruch 11 oder 12, **dadurch gekennzeichnet, dass** die Aufsatzvorrichtung (300) eine Rückstelleinrichtung (402) umfasst, entgegen deren Wirkung das dritte Kupplungsglied (380) mit dem vierten Kupplungsglied (384) in Wirkverbindung bringbar ist.

14. Aufsatzvorrichtung nach Anspruch 12 oder 13 , **dadurch gekennzeichnet, dass** das vierte Kupplungsglied (384) mit der ersten Kupplungseinrichtung (354) mechanisch gekoppelt ist.

15. Chirurgische Vorrichtung (11; 101; 201; 301) zum Durchschneiden eines Knochens mit mindestens einer Aufsatzvorrichtung (10; 100; 200; 300), wobei die mindestens eine Aufsatzvorrichtung (10; 100; 200; 300) gemäß einem der Ansprüche 1 bis 14 ausgebildet ist.

## Claims

1. Attachment device (10; 100; 200; 300) for a surgical device, the attachment device (10; 100; 200; 300) comprising a cutting device (12) with at least one cutting element (22) forming at least one cutting edge (26) for producing at least one cut line on a bone (88), the attachment device (10; 100; 200; 300) comprising a feed device (34) having at least one feed member (62) which is configured for movement relative to this at least one cutting edge (26) and can thereby assume a variable spacing relative thereto, and comprising a force application device (48) which is configured such that the at least one feed member (62) can have a force acting in the distal direction applied thereto, at least insofar as the spacing exceeds a minimum spacing, and the attachment device (200; 300) comprising a support device (202) with at least one support member (206) which can be made to bear against the bone (88), **characterized in that** the support device (202) is configured so as to be secured against rotation relative to the feed device (34).

2. Attachment device in accordance with claim 1, **characterized in that** the at least one feed member (62) comprises at least one engagement element (68) extending in the distal direction for engagement in the bone (88).

3. Attachment device in accordance with claim 1 or 2, **characterized in that** the feed device (34) is configured so as to be rotationally movable relative to the cutting device (12).

4. Attachment device in accordance with any one of the preceding claims, **characterized in that** the at least one feed member (62) is configured so as to be secured against rotation relative to the feed device (34).

5. Attachment device in accordance with any one of the preceding claims, **characterized in that** the cutting device (12) forms a screw connection with the feed device (34).

6. Attachment device in accordance with any one of the preceding claims, **characterized in that** the support device (202) is configured so as to be rotationally movable relative to the cutting device (12).

7. Attachment device in accordance with any one of the preceding claims, **characterized in that** the support device (202) forms a screw connection with the cutting device (12).

8. Attachment device in accordance with any one of the preceding claims, **characterized in that** the attachment device (300) comprises a first coupling device (354), associated with the cutting device (12), with a first coupling member (346) which is in operative connection with the at least one cutting element (22) and a drivable second coupling member (350) which interacts with the first coupling member (346).

9. Attachment device in accordance with claim 8, **characterized in that** the first coupling member (346) can be brought into operative connection with the second coupling member (350) by the application of force to the at least one cutting element (22) and/or to the at least one feed member (34) in the proximal direction.

10. Attachment device in accordance with claim 8 or 9, **characterized in that** the attachment device (300) comprises a readjusting device (344) against the action of which the first coupling member (346) can be brought into operative connection with the second coupling member (350).

11. Attachment device in accordance with any one of the preceding claims, **characterized in that** the attachment device (300) comprises a second coupling device (386), associated with the support device (202), with a third coupling member (380) which is in operative connection with the at least one support member (206) and a drivable fourth coupling member (384) which interacts with the third coupling member (380).

12. Attachment device in accordance with claim 11, **characterized in that** the third coupling member (380) can be brought into operative connection with the fourth coupling member (384) by the application of force to the at least one support member (206) in the proximal direction.

13. Attachment device in accordance with claim 11 or 12, **characterized in that** the attachment device (300) comprises a readjusting device (402) against the action of which the third coupling member (380) can be brought into operative connection with the fourth coupling member (384).

14. Attachment device in accordance with claim 12 or 13, **characterized in that** the fourth coupling member (384) is mechanically coupled to the first coupling device (354).

15. Surgical device (11; 101; 201; 301) for cutting through a bone with at least one attachment device (10; 100; 200; 300), the at least one attachment device (10; 100; 200; 300) being configured in accordance with any one of claims 1 to 14.

## Revendications

1. Dispositif rapporté (10; 100; 200; 300) pour un dispositif chirurgical, le dispositif rapporté (10; 100; 200; 300) comprenant un dispositif de coupe (12) avec au moins un élément de coupe (22) formant au moins une arête de coupe (26) pour produire au moins une ligne de coupe sur un os (88), le dispositif rapporté (10; 100; 200; 300) comportant un dispositif d'avance (34) avec au moins un organe d'avance (62), qui est réalisé mobile par rapport à cette au moins une arête de coupe (26) en pouvant ainsi présenter une distance variable par rapport à celle-ci, ainsi qu'un dispositif d'application de force (48), qui est conçu de manière à ce que ledit au moins un organe d'avance (62) puisse être sollicité par une force agissant en direction distale, tout au moins dans la mesure où ladite distance dépasse une distance minimale, et le dispositif rapporté (200; 300) comprenant un dispositif d'appui (202) avec au moins un organe d'appui (206) pouvant être appliqué contre l'os (88), **caractérisé en ce que** le dispositif d'appui (202) est réalisé sécurisé en rotation par rapport au dispositif d'avance (34).

2. Dispositif rapporté selon la revendication 1, **caractérisé en ce que** ledit au moins un organe d'avance (62) comporte au moins un élément d'engagement (68) s'étendant dans la direction distale et destiné à venir mordre dans l'os (88).

3. Dispositif rapporté selon la revendication 1 ou la revendication 2, **caractérisé en ce que** le dispositif d'avance (34) est réalisé mobile en rotation par rapport au dispositif de coupe (12).

4. Dispositif rapporté selon l'une des revendications précédentes, **caractérisé en ce que** ledit au moins un organe d'avance (62) est réalisé sécurisé en rotation par rapport au dispositif d'avance (34).

5. Dispositif rapporté selon l'une des revendications précédentes, **caractérisé en ce que** le dispositif de coupe (12) forme avec le dispositif d'avance (34), une liaison par vissage.

6. Dispositif rapporté selon l'une des revendications précédentes, **caractérisé en ce que** le dispositif d'appui (202) est réalisé mobile en rotation par rapport au dispositif de coupe (12).

7. Dispositif rapporté selon l'une des revendications précédentes, **caractérisé en ce que** le dispositif d'appui (202) forme avec le dispositif de coupe (12), une liaison par vissage.

8. Dispositif rapporté selon l'une des revendications précédentes, **caractérisé en ce que** le dispositif rapporté (300) présente un premier dispositif de couplage (354) associé au dispositif de coupe (12) et comprenant un premier organe de couplage (346) en liaison d'interaction avec ledit au moins un élément de coupe (22), ainsi qu'un deuxième organe de couplage (350) pouvant être entraîné et interagissant avec le premier organe de couplage (346).

9. Dispositif rapporté selon la revendication 8, **caractérisé en ce que** le premier organe de couplage (346) peut être amené en liaison d'interaction avec le deuxième organe de couplage (350), lorsque ledit au moins un élément de coupe (22) et/ou ledit au moins un organe d'avance (34) sont sollicités par une force dans la direction proximale.

10. Dispositif rapporté selon la revendication 8 ou la revendication 9, **caractérisé en ce que** le dispositif rapporté (300) comprend un dispositif de rappel (344) à l'encontre de l'action duquel le premier organe de couplage (346) peut être amené en liaison d'interaction avec le deuxième organe de couplage (350).

11. Dispositif rapporté selon l'une des revendications précédentes, **caractérisé en ce que** le dispositif rapporté (300) présente un deuxième dispositif de couplage (386) associé au dispositif d'appui (202) et comprenant un troisième organe de couplage (380) en liaison d'interaction avec ledit au moins un organe d'appui (206), ainsi qu'un quatrième organe de couplage (384) pouvant être entraîné et interagissant avec le troisième organe de couplage (380).

12. Dispositif rapporté selon la revendication 11, **caractérisé en ce que** le troisième organe de couplage (380) peut être amené en liaison d'interaction avec le quatrième organe de couplage (384), lorsque ledit au moins un organe d'appui (206) est sollicité par une force dans la direction proximale.

13. Dispositif rapporté selon la revendication 11 ou la revendication 12, **caractérisé en ce que** le dispositif rapporté (300) comprend un dispositif de rappel (402) à l'encontre de l'action duquel le troisième organe de couplage (380) peut être amené en liaison d'interaction avec le quatrième organe de couplage (384).

14. Dispositif rapporté selon la revendication 12 ou la revendication 13, **caractérisé en ce que** le quatrième organe de couplage (384) est accouplé mécaniquement au premier dispositif de couplage (354).

15. Dispositif chirurgical (11; 101; 201; 301) pour sectionner un os par coupe, comprenant au moins un dispositif rapporté (10; 100; 200; 300), ledit au moins un dispositif rapporté (10; 100; 200; 300) étant configuré selon l'une des revendications 1 à 14.
